# EUROPÄISCHE PATENTSCHRIFT

(11) **EP 2 848 882 B1**
(45) Veröffentlichungstag und Bekanntmachung des Hinweises auf die Patenterteilung: **09.03.2016**
(21) Anmeldenummer: 14189484.0
(22) Anmeldetag: 02.05.2013
(51) Int. Cl.: F26B 5/06, F26B 25/00, B67B 3/10, B67B 3/14

(54) **Verfahren und Vorrichtung zum Bördeln von Behältern zur Aufbewahrung von Substanzen für medizinische, pharmazeutische oder kosmetische Anwendungen**
Method and device for crimping containers for the storage of substances for medical, pharmaceutical or cosmetic uses
Procédé et dispositif de sertissage de récipients pour la conservation de substances destinées à des applications médicales, pharmaceutiques ou cosmétiques

(30) Priorität: 03.05.2012 DE 102012103899; 03.05.2012 US 201261642125 P; 13.07.2012 DE 102012106341; 04.09.2012 DE 102012108215; 04.09.2012 US 201261696457 P; 05.11.2012 DE 102012110547
(43) Veröffentlichungstag der Anmeldung: 18.03.2015
(62) Teilanmeldung aus: 13720925.0
(73) Patentinhaber: Schott AG, 55122 Mainz (DE)
(72) Erfinder: Wissner, Kai, 69493 Hirschberg (DE); Pawlowski, Edgar, 55271 Stadecken-Elsheim (DE); Deutschle, Gregor Fritz, 65185 Wiesbaden (DE); Koch, Kristophe, Lebanon, 17042 (US)
(74) Vertreter: 2K Patentanwälte Blasberg Kewitz & Reichel

(56) Entgegenhaltungen:
- DE-A1- 3 908 582
- DE-A1-102009 027 452
- GB-A- 295 672

## Beschreibung

### Gebiet der Erfindung

Die vorliegende Erfindung betrifft allgemein die gleichzeitige Behandlung oder Weiterverarbeitung von Behältern, die zur Aufbewahrung von Substanzen für kosmetische, medizinische oder pharmazeutische Anwendungen dienen oder diese enthalten sollen, insbesondere von Fläschchen mit Wirkstoffen oder Wirkstofflösungen, und betrifft insbesondere ein Verfahren und eine Vorrichtung zum Bördeln von solchen Behältern.

### Hintergrund der Erfindung

Als Behälter zur Aufbewahrung und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen, Ampullen oder Karpullen, eingesetzt. Diese weisen generell eine zylindrische Form auf, können aus Kunststoffen oder aus Glas hergestellt werden und sind kostengünstig in großen Mengen erhältlich. Für eine möglichst wirtschaftliche Befüllung der Behälter unter sterilen Bedingungen werden in zunehmendem Maße Konzepte eingesetzt, bei denen die Behälter gleich beim Hersteller der Behälter in Transport- und Verpackungsbehälter steril verpackt werden, die bei einem Pharmaunternehmen dann unter sterilen Bedingungen, insbesondere in einem sog. Steriltunnel, ausgepackt und dann weiter verarbeitet werden.

Zu diesem Zweck sind aus dem Stand der Technik div. Transport- und Verpackungsbehälter bekannt, in denen gleichzeitig eine Mehrzahl von Medikamentenbehältern in einer regelmäßigen Anordnung, beispielsweise in einer Matrixanordnung entlang von Reihen und sich rechtwinklig dazu erstreckenden Spalten, angeordnet sind. Dies hat Vorteile bei der automatisierten Weiterverarbeitung der Behälter, da die Behälter an kontrollierten Positionen und in vorgegebener Anordnung an Bearbeitungsstationen übergeben werden können, beispielsweise an Prozessautomaten, Roboter oder dergleichen. Hierzu werden Haltestrukturen eingesetzt, in welchen gleichzeitig eine Mehrzahl von Behältern in einer vorbestimmten regelmäßigen Anordnung gehalten werden können. Zur Übergabe an eine Bearbeitungsstation braucht einfach nur der Transport- und Verpackungsbehälter geeignet positioniert und geöffnet zu werden. Die nachgeordnete Bearbeitungsstation weiß dann, in welcher Position und Anordnung die weiter zu verarbeitenden Behälter angeordnet sind.

Ein solcher Transport- und Verpackungsbehälter und ein entsprechendes Verpackungskonzept sind beispielsweise in der US 8,118,167 B2 offenbart. Die Weiterverarbeitung der Behälter erfolgt jedoch stets in der Weise, dass die Haltestruktur aus dem Transport- und Verpackungsbehälter entnommen und die Behälter aus der Haltestruktur entnommen und vereinzelt werden und auf einer Fördereinrichtung, insbesondere einem Förderband, einzeln an die Bearbeitungsstationen übergeben und dort weiterverarbeitet werden. Dies begrenzt die erzielbare Geschwindigkeit bei der Weiterverarbeitung. Insbesondere bei der Vereinzelung der Behälter mit Hilfe von Zellenrädern oder dergleichen kommt es immer wieder dazu, dass einzelne Behälter unkontrolliert aneinanderstoßen, was zu einem unerwünschten Abrieb und in der Folge zu einer Verunreinigung des Behälterinnenraums oder der Prozessanlage sowie zu einer Beeinträchtigung des äußeren Erscheinungsbildes der Behälter führt, was unerwünscht ist.

US 8,100,263 B2 offenbart einen steril verpackbaren und transportierbaren Transport- und Verpackungsbehälter, in welchen eine plattenförmige Haltestruktur eingesetzt werden kann, in der eine Mehrzahl von Medikamentenbehältern in einer regelmäßigen Anordnung gehalten werden. Die Medikamentenbehälter können nicht in dem Transport- oder Verpackungsbehälter oder in der Haltestruktur weiter verarbeitet werden, sondern müssen zunächst in der herkömmlichen Weise vereinzelt und an nachgeordnete Bearbeitungsstationen übergeben werden.

Weitere vergleichbare Transport- und Verpackungsbehälter und Haltestrukturen werden in WO 2011/135085 A1, US 2011/0277419 A1, WO 2012/025549 A1, WO 2011/015896 A1, WO 2012/007056 A1 und WO 2009/015862 A1 offenbart.

Zur Weiterverarbeitung müssen die Medikamentenbehälter jedoch stets vereinzelt werden. Eine chargenweise Weiterverarbeitung von Medikamentenbehälter ist herkömmlich nicht möglich.

DE 10 2009 027 452 A1 offenbart eine Vorrichtung zum Füllen und Verschließen von pharmazeutischen Behältnissen gemäß dem Oberbegriff des Anspruchs 15, sowie ein Verfahren zur Behandlung von Behältern gemäß dem Oberbegriff des Anspruchs 1. Eine Mehrzahl von Behältnissen ist in einem Trägerelement angeordnet, dass zur weiteren Verarbeitung zunächst einem Transportbehälter entnommen wird. Zum Bördeln werden die Behältnisse aus dem Trägerelement einzeln herausgenommen, mit einem Metalldeckel versehen und dieser wird auf den oberen Rand der Behältnisse gebördelt. Anschließend werden die Behältnisse wieder in das Trägerelement eingesetzt und dieses in einen Transportbehälter eingesetzt. Die Behältnisse werden also nicht chargenweise gebördelt.

DE 39 08 582 A1 offenbart eine Vorrichtung zum Schließen von Flaschen, bei der die Flaschen zum Bördeln auf einem Drehteller angehoben werden. Die Flaschen werden jedoch der Vorrichtung einzeln zugeführt.

GB 295 672 A offenbart offenbart Flaschen, welche in einem Träger (aus Drahtgeflecht) angeordnet sind und zum Verschließen in Reihen in der jeweiligen Aufnahme des Träger in eine angehobene Position verschoben sind. Die Behälter werden jedoch nicht gebördelt. Auch erlaubt die Art des Trägers konstruktiv keine Anhebung mittels Drehteller.

### Zusammenfassung der Erfindung

Aufgabe der vorliegenden Erfindung ist es, ein verbessertes Verfahren zur Behandlung oder Verarbeitung von Behältern, die zur Aufbewahrung von Substanzen fur kosmetische, medizinische oder pharmazeutische Anwendungen dienen, bereitzustellen, bei dem die Behälter gebördelt werden und das noch rascher und wirtschaftlicher, besser automatisierbar und zuverlässiger ausgeführt werden kann. Gemäß einem weitern Gesichtspunkt der vorliegenden Erfindung soll eine entsprechend ausgelegte Vorrichtung bereitgestellt werden.

Diese Aufgabe wird gemäß der vorliegenden Erfindung durch ein Verfahren mit den Merkmalen nach Anspruch 1 sowie durch eine Vorrichtung nach Anspruch 15 gelöst. Weitere vorteilhafte Ausführungsformen sind Gegenstand der rückbezogenen Unteransprüche.

Bei einem Verfahren zur Behandlung oder Verarbeitung von Behältern, die zur Aufbewahrung von Substanzen für kosmetische, medizinische oder pharmazeutische Anwendungen dienen oder diese enthalten, insbesondere von Fläschchen, werden die Behälter mittels einer Fördereinrichtung zur Behandlung oder Verarbeitung automatisiert an Prozessstationen vorbeigeführt oder durchlaufen diese, wobei eine Mehrzahl von Behältern von der Fördereinrichtung gefördert wird, während diese von einem Träger gemeinsam in einer regelmäßigen zweidimensionalen Anordnung gehalten werden, wobei der Träger eine Mehrzahl von Öffnungen oder Aufnahmen aufweist, welche die regelmäßige Anordnung vorgeben, und die Behandlung oder Verarbeitung der Behälter an oder in zumindest einer der Prozessstationen ausgeführt wird, während diese von dem Träger gehalten werden oder in Öffnungen oder Aufnahmen des Trägers aufgenommen sind. Bei dem Verfahren wird mittels einer Bördeleinrichtung auf dem oberen Rand der Behälter ein Metalldeckel gebördelt, wobei die Behälter zum Bördeln in der jeweiligen Öffnung oder Aufnahme in Längsrichtung in eine angehobene Position verschoben werden und in der angehobenen Position von einem Drehteller um ihre Längsachse gedreht werden, während die Behälter in den Öffnungen oder Aufnahmen des Trägers aufgenommen sind, und nach dem Bördeln wieder in die Öffnungen oder Aufnahmen zurückgeschoben werden, um von dem Träger gehalten zu werden.

Somit können die Behälter erfindungsgemäß chargenweise behandelt oder verarbeitet werden. Einer die Prozesse aufwändig machenden Entnahme aus dem Träger und einer Vereinzelung bedarf es erfindungsgemäß nicht. Hierzu ist der Träger erfindungsgemäß so ausgelegt, dass die Behälter an diesem form- oder reibschlüssig gehalten werden können. Insbesondere werden die Behälter in hierfür geeignet ausgebildeten Öffnungen oder Aufnahmen des Trägers gehalten.

Bevorzugt sind Seitenwandabschnitte und/oder der Boden der Behälter, während diese an dem Träger gehalten werden, jedenfalls größtenteils zugänglich, sodass die Behälter an dem Träger einfach gehandhabt werden können. Beispielsweise kann der Boden der Behälter vollständig oder zum überwiegenden Teil zugänglich, also nicht von einer Haltestruktur oder dergleichen abgedeckt, sein, während diese an dem Träger gehalten werden. Damit können die Träger mit den Behältern beispielsweise auf einem Kühlboden eines Gefriertrockenschranks abgesetzt werden, sodass ein vollflächiger Kontakt für eine effiziente Kühlung gewährleistet ist. Zweckmäßig sind hierzu sämtliche Behälter an dem Träger auf "gleicher Höhe" gehalten.

Die Träger sind erfindungsgemäß so ausgelegt sein, dass die Behälter, während diese an diesen gehalten werden, verschoben oder gedreht oder in vergleichbarer Weise verstellt oder bewegt werden können. Dies lässt sich durch entsprechende Auslegung des Form- oder Reibschlusses in einfacher Weise gewährleisten. Somit können die Behälter beispielsweise zum Bördeln eines Metalldeckels, der auf deren oberen Rand aufgesetzt ist, gedreht werden, während diese an dem Träger gehalten sind.

Erfindungsgemäß können die Behälter zur Behandlung oder Verarbeitung an oder in der Prozessstation insbesondere in der jeweiligen Öffnung oder Aufnahme des Trägers in Längsrichtung in eine angehobene Position verschoben werden, in welcher die weitere Behandlung oder Verarbeitung dann erleichtert ist. Beispielsweise kann in dieser angehobenen Position der Boden der Behälter vollständig zugänglich sein oder der obere Rand der Behälter über den oberen Rand des Trägers oder eines Transport- und Verpackungsbehälters geeignet weit vorstehen, sodass erst in der angehobenen Position eine Behandlung oder Verarbeitung möglich ist.

Zweckmäßig werden die Behälter in dieser angehobenen Position im Bereich Ihrer zylindrischen Seitenwand oder aber eines verengten Halsabschnitts unterhalb des oberen Rands oder an ihrem oberen Rand gehalten, was von.der jeweiligen Prozessstation abhängen kann.

Gemäß einer weiteren Ausführungsform sind die Behälter in der angehobenen Position weiterhin in den Öffnungen oder Aufnahmen des Trägers aufgenommen, werden jedoch auf einer zusätzlichen Abstützfläche abgestützt oder mittels einer zusätzlichen Halte- oder Greifeinrichtung gehalten, während diese man oder in der Prozessstation behandelt oder verarbeitet werden. Die Haltemittel an dem Träger sind also so ausgelegt, dass diese in der angehobenen Position die Behälter nicht halten, jedenfalls nicht mit einer dem Gewicht der Behälter entsprechenden ausreichenden Haltekraft. Jedoch brauchen auch bei dieser Ausführungsform die Behälter nicht vollständig aus dem Träger entnommen zu werden, sodass diese weiterhin chargenweise behandelt oder verarbeitet werden können, jedoch dennoch rascher an einen nachfolgenden Prozessschritt übergeben werden können. Bei der vorgenannten Halte- oder Greifeinrichtung kann es sich beispielsweise um einen Roboterarm einer volllautomatisch gesteuerten Prozessanlage handeln.

Bei der vorgenannten Abstützfläche kann es sich insbesondere auch um Führungsflächen handeln, die die weitere Förderung der Behälter durch die Prozessanlage geeignet fuhren. Diese Führungsflächen können beispielsweise auch kurven- oder rampenförmig ausgebildet sein, um so Höhenlagen der Behälter während ihrer Förderung durch die Prozessanlage geeignet vorzugeben. Insbesondere kann eine solche Abstützfläche auch mit einem Drehteller versehen oder als solcher ausgebildet sein, um einzelne Behälter zu drehen, während diese auch weiterhin in den Öffnungen oder Aufnahmen des Trägers aufgenommen sind. Zweckmäßig ist hierzu der Träger so ausgelegt, dass die von den Haltemitteln ausgeübte Haltekraft in einfacher Weise verstellt werden kann, nämlich zwischen einer ersten Haltestellung, in der die Behälter mit ausreichender Kraft form- oder reibschlüssig an dem Träger gehalten sind, und einer zweiten Haltestellung, in der die Haltekraft vollständig oder jedenfalls in ausreichendem Maße reduziert ist. Dies kann beispielsweise durch Verstellung der Öffnungsweite der Öffnungen oder Aufnahmen des Trägers in einfacher Weise bewerkstelligt werden.

Gemäß einer weiteren Ausführungsform können Randabschnitte des Trägers, insbesondere einer Grundplatte desselben, abgenommen oder weggeschwenkt werden, um die Grundfläche des Trägers insgesamt zu verringern, wenn die Behälter in oder an der Prozessstation behandelt oder verarbeitet werden. Dies führt insbesondere bei der Gefriertrocknung einer Mehrzahl von in einem Träger gehaltenen Behältern zu bedeutenden Kosteneinsparungen.

Gemäß einem ersten Gesichtspunkt der vorliegenden Erfindung sind die Behälter (container) in dem Träger (Haltestruktur) reibschlüssig gehalten. Zur kraft- bzw. reibschlüssigen Halterung von zylindrischen Behältern steht eine Vielzahl von Haltemitteln zur Verfügung. Kraft- bzw. reibschlüssige Verbindungen setzen bekanntermaßen nur eine ausreichende NormalKraft auf die miteinander zu verbindenden Flächen voraus. Die gegenseitige Verschiebung zwischen Behälter und Träger ist also verhindert solange die durch die Haftreibung zwischen Träger und Behälter bewirkte Gegenkraft nicht überschritten wird. Der Kraft- bzw. Reibschluss ist verloren und die Flächen rutschen aufeinander, wenn die tangential wirkende Last-Kraft größer als die Haftreibungs-Kraft ist. Letzteres ist jedoch bei den vergleichsweise geringen Gewichten der in dem Träger aufzunehmenden Behälter unwahrscheinlich, kann jedoch ausgenutzt werden, um die Behälter, während diese in dem Träger aufbewahrt sind, von einer ersten Stellung axial in eine zweite Stellung zu verschieben, in welcher diese weiterverarbeitet werden können, beispielsweise deren Öffnungen mit einem Stopfen verschlossen werden oder auf den Stopfen ein äußerer Verschluss (beispielsweise Bördelkappe oder Krampe), häufig aus Aluminiumblech, aufgebracht wird.

Der Kraft- bzw. Reibschluss erfolgt zweckmäßig entweder unterhalb des aufgeweiteten oberen Randes der Behälter, d.h. an ihrem verengten Hals- bzw. Nackenbereich unterhalb des oberen Rands, oder im Bereich der zylindrischen Seitenwand. Einer bodenseitigen Abstützüng der Behälter bedarf es erfindungsgemäß grundsätzlich nicht, sodass ein Zugriff auf die Unterseiten (Böden) der in dem Träger gehaltenen Behälter grundsätzlich möglich ist. Dies ermöglicht erfindungsgemäß, dass die Behälter, während diese in dem Träger aufgenommen sind, weiterverarbeitet werden können, wie nachfolgend ausgeführt. Mit anderen Worten, die Behälter können in Trägern chargenweise weiter verarbeitet werden, bleiben jedoch während der Weiterverarbeitung weiterhin zuverlässig und kollisionsfrei in oder an den Trägern gehalten, was erhebliche Geschwindigkeitsvorteile und Vorteile bei der Automatisierung von Weiterverarbeitungsanlagen ermöglicht und so insgesamt zu noch wirtschaftlicheren und kostengünstigeren Prozessen führt. Ferner ist ein unmittelbarer Glas-Glas-Kontakt von benachbarten Behältern zuverlässig verhindert, was Abrieb und Verunreinigungen innerhalb der Weiterverarbeitungsanlage wirkungsvoll verhindert und so erheblich längeren Laufzeiten und Wartungsintervalle der Anlagen ermöglicht. Ferner können Kratzer oder die Erzeugung von Partikeln auf oder in den Behältern wirkungsvoll verhindert werden.

Der erfindungsgemäße Träger ermöglicht dabei zweckmäßig die Entnahme der Behälter nach oben oder unten. Da die Position des Kraft- bzw. Reibschlusses zwischen Behälter und Haltestruktur einfach variiert werden kann, ist die erfindungsgemäße Haltestruktur sehr flexibel auch für Behälter mit unterschiedlichen Außenabmessungen einsetzbar, solange eine ausreichende Normalkraft für den Reibschluss gewährleistet werden kann. Insbesondere können die Behälter in dem Träger in axialer Richtung einfach verschoben werden, sodass Behälter mit unterschiedlichen Höhen in oder an demselben Träger gehalten werden können. Die axiale Verschieblichkeit der Behälter ermöglicht auch einen einfachen Toleranzausgleich.

Gemäß einem zweiten Gesichtspunkt der vorliegenden Erfindung sind die Behälter (container) in der Haltestruktur formschlüssig gehalten. Zur formschlüssigen Halterung von zylindrischen Behältern steht eine Vielzahl von Haltemitteln zur Verfügung. Die gegenseitige Verschiebung zwischen Behälter und Haltestruktur ist verhindert, solange ein Verbindungspartner dem anderen Verbindungspartner im Wege steht, diesen also sperrt.

Der Formschluss erfolgt zweckmäßig entweder unterhalb des aufgeweiteten oberen Randes der Behälter, d.h. im Bereich des verengten Hals- bzw. Nackenbereichs und unmittelbar unterhalb des oberen Rands, oder am unteren Ende der Behälter, beispielsweise an einem Boden der Behälter. Zweckmäßig ist der aufgeweitete obere Rand oder das untere Ende bzw. der Boden der Behälter auf den Formschlusselementen der Haltestruktur unmittelbar abgestützt. Alternativ kann der obere Rand oder das untere Ende bzw. der Boden der Behälter auch formschlüssig umgriffen oder hintergriffen werden.

Gemäß einer weiteren Ausführungsform wird der vorgenannte Formschluß insbesondere mittels Haltemitteln ausgebildet, wobei als Haltemittel zumindest zwei Haltezungen an dem Träger bzw. der Haltestruktur vorgesehen sind, die am Rand einer jeweiligen Öffnung oder Aufnahme vorgesehen sind und von einer Oberseite des Trägers abragen, um den jeweiligen Behälter in der Öffnung oder Aufnahme zu halten. Dabei sind die Haltezungen so ausgelegt, dass diese beim Einführen der Behälter in die Öffnungen oder Aufnahmen elastisch weggeschwenkt oder weggeklappt werden, und ferner so auf die Behälter abgestimmt, dass diese mit radialem Spiel von den Haltezungen gehalten sind. Das radiale Spiel ermöglicht, dass Behälter mit unterschiedlichen radialen Toleranzen und/oder Außenabmessungen von derselben Haltestruktur zuverlässig gehalten werden können. Das radiale Spiel ist zweckmäßig so ausgelegt und auf die Außenkontur und -abmessung der Behälter abgestimmt, dass niemals gleichzeitig sämtliche Haltezungen den verengten Halsabschnitt am oberen Ende der Behälter, insbesondere Fläschchen, berühren. Gleichzeitig verhindert das radiale Spiel auch ein unerwünschtes Verspannen oder gar Aufwölben des Trägers beim Halten von Behältern mit unterschiedlichen radialen Toleranzen und/oder Außenabmessungen, was erhebliche Vorteile insbesondere bei der gleichzeitigen Prozessierung einer Mehrzahl von Behältern, während diese von der Haltestruktur gehalten sind, bietet, beispielsweise bei der Gefriertrocknung bei Prozessierung bei sehr niedrigen Temperaturen.

Selbst wenn sich der Träger dennoch bei der Prozessierung verziehen oder aufwölben sollte, kann dennoch ein gleichmäßiger Bodenkontakt zu sämtlichen von der Haltestruktur gehaltenen Behältern realisiert werden, insbesondere wenn diese ergänzend mit einem ausreichendem axialen Spiel von den Haltezungen an der Haltestruktur gehalten sind, da das axiale Spiel zusätzlich auch einen Längentoleranzausgleich ermöglicht.

Die Haltezungen sind dabei ausreichend elastisch ausgebildet oder gelagert, sodass die Behälter axial, d.h. in Richtung der Längsachse der Behälter und senkrecht zur Ebene des Trägers, von der Ober- oder Unterseite des Trägers her in die Öffnungen oder Aufnahmen eingeschoben werden können, insbesondere unter elastischer Verformung der Haltezungen, beispielsweise unter Wegbiegen derselben. Die Bestückung des Trägers mit Behältern kann somit einfach automatisiert werden, was durch eine regelmäßige Anordnung der Öffnungen oder Aufnahmen, bevorzugt in einer zweidimensionalen Matrix, noch weiter begünstigt wird.

Als bevorzugte Stelle, an der die Behälter an den Haltezungen gehalten oder abgestützt sind, hat sich die Unterseite eines verbreiterten oberen Randabschnittes der Behälter erwiesen, wie diese insbesondere bei Fläschchen typischerweise als sog. Rollrand oder Schulter vorgesehen sind. In diesem Bereich steht eine Abstütz- oder Lagerfläche zum Halten oder Abstützen der Behälter mit einer ausreichenden Erstreckung in Radialrichtung der Öffnungen oder Aufnahmen zur Verfügung, um das vorgenannte radiale Spiel bei der Halterung der Behälter ohne Weiteres zu realisieren.

Weil die Behälter in den Öffnungen oder Aufnahmen mit sehr geringem Kraftaufwand angehoben oder bewegt, beispielsweise gedreht, werden können, können diese, während diese sich in der Haltestruktur befinden und von dieser gehalten oder zumindest geführt werden, ohne weiteres bearbeitet werden. Als besonders vorteilhaft hat sich diese Art der Halterung z.B. beim Verschließen der Behälter durch Bördeln eines Metalldeckels erwiesen. Die hierzu erforderlichen Vorgänge können an dem Metalldeckel ausgeführt werden, während der Behälter in der Öffnung oder Aufnahme der Haltestruktur gehalten oder zumindest geführt ist. Als besonders vorteilhaft hat sich diese Art der Halterung auch bei der Prozessierung von Behältern erwiesen, während diese in der Haltestruktur gehalten bzw. aufgenommen sind. Beispielsweise können die Haltestrukturen mit den darin aufgenommenen bzw. gehaltenen Behältern in einen Gefriertrockenschrank eingebracht werden. Aufgrund der Halterung der Behälter mit einem gewissen Spiel in den Haltestrukturen kann gewährleistet werden, dass die Böden von sämtlichen Behältern auf einer kühlenden Unterlage, beispielsweise einem Kühlfinger des Gefriertrockenschranks, gleichmäßig aufliegen. Oder die Behälter können ohne größeren Kraftaufwand in den Öffnungen oder Aufnahmen der Haltestruktur angehoben und zur Prozessierung gehandhabt werden.

Gemäß einer bevorzugten Ausführungsform sind die Haltezungen als elastische Haltezungen ausgebildet, verfügen jedoch über eine ausreichende Elastizität, um beim Einführen der Behälter in die Öffnungen oder Aufnahmen ausreichend elastisch weggeschwenkt oder weggeklappt zu werden, um den Behältern den Weg in die Öffnungen oder Aufnahmen freizugeben. Dies lässt sich durch geeignete Dimensionierung, Materialwahl und Auslegung der Materialstärke der Haltezungen ohne weiteres erreichen. Bevorzugt sind die Haltezungen somit aus einem Kunststoff ausgebildet.

Gemäß einer Ausführungsform sind die Haltezungen elastisch gegen eine Haltestellung vorgespannt, bevorzugt mittels eines elastischen Rückstellelements, beispielsweise einer Rückstellfeder oder eines Kunststoffblättchens oder elastischen Kunststoffgebildes, das mit der zugeordneten Haltezunge geeignet zusammenwirkt und auf der Oberseite des Trägers vorgesehen oder ausgebildet ist.

Gemäß einer Ausführungsform sind die Haltezungen so auf die Behälter abgestimmt, dass die Behälter mit einem verbreiterten Rand, der an einem oberen Ende der Behälter ausgebildet ist, also insbesondere mit dem vorgenannten Rollrand, lose auf Oberseiten der Haltezungen aufliegen. Die Behälter können somit ohne Widerstand nach oben wieder aus den Öffnungen oder Aufnahmen entnommen werden.

Gemäß einer Ausführungsform umgreifen die Haltezungen den verbreiterten Rand dergestalt, dass die Behälter mit radialem Spiel oder mit radialem und axialem Spiel von den Haltezungen gehalten sind. Auf diese Weise können die Behälter in den Öffnungen oder Aufnahmen axial verliersicher gehalten werden. Zum Entnehmen der Behälter aus den Öffnungen oder Aufnahmen brauchen die Haltezungen nur wiederum in der Art, wie beim Einführen der Behälter, zurückgeschwenkt oder zurück geklappt werden.

Gemäß einer Ausführungsform sind die Haltezungen so verteilt auf der Oberseite des Trägers angeordnet, dass diese beim Wegschwenken oder Wegklappen einander nicht unmittelbar berühren und eine unmittelbar benachbarte Öffnung oder Aufnahme nicht versperren. Somit kann die Packungsdichte der Behälter an dem Träger noch weiter erhöht werden. Insbesondere sind die Haltezungen so ausgelegt, dass unmittelbar benachbarte Haltezungen, wenn diese beim Einführen der Behälter in die zugeordneten Öffnungen oder Aufnahmen zum Träger hin geschwenkt oder geklappt werden, einander nicht berühren.

Gemäß einer Ausführungsform sind am oberen Ende der Haltezungen Einführschrägen ausgebildet, welche jeweils in eine von den Haltezungen radial einwärts vorstehende Haltenase zum Halten der Behälter übergehen. Die Behälter können somit noch einfacher und kraftärmer in die Öffnungen oder Aufnahmen eingeführt werden. Insbesondere geraten beim Einführen der Behälter von oben her in die Öffnungen oder Aufnahmen zunächst die Böden bzw. unteren Enden der Behälter in Anlage zu den Einführschrägen. Beim weiteren Einführen der Behälter gleitet das untere Ende bzw. der Boden der Behälter entlang den Einführschrägen abwärts und spreizt dabei die Haltezungen auseinander oder klappt bzw. schwenkt diese zurück. Beim weiteren Einführen der Behälter gerät schließlich die zylindrische Seitenwand in Anlage zu den Haltenasen und gleitet an diesen entlang, solange bis schließlich die Unterseite des vorgenannten Rollrands lose auf den Haltenasen der Haltezungen aufliegt.

Ein weiterer Gesichtspunkt der vorliegenden Erfindung betrifft ferner einen Transport- und Verpackungsbehälter mit zumindest einer Haltestruktur, wie vorstehend ausgeführt und nachfolgend weiter im Detail offenbart.

Ein weiterer Gesichtspunkt der vorliegenden Erfindung betrifft ferner einen Transport- und Verpackungsbehälter mit Maßnahmen für einen Plagiatsschutz, insbesondere für eine Identifikation und/oder Nachverfolgung, wie nachfolgend ausgeführt.

### Figurenübersicht

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1a - 1c: einen Transport- und Verpackungsbehälter gemäß einer ersten Ausführungsform zur Verwendung bei einem Verfahren gemäß der vorliegenden Erfindung;
- Fig. 2a - 2d: eine Prozessstation zum Bördeln eines Metalldeckels auf einem oberen Rand einer Mehrzahl von Behältern bei einem Verfahren gemäß der vorliegenden Erfindung;
- Fig. 2e: in einer vergrößerten Teilansicht die Vorgehensweise zum Bördeln eines Metalldeckels auf einem oberen Rand von Behältern bei der Prozessstation gemäß den Fig. 2a bis 2d;
- Fig. 2f und 2g: zwei weitere Varianten für Prozessstationen zum Bördeln eines Metalldeckels auf einem oberen Rand einer Mehrzahl von Behältern bei einem Verfahren gemäß der vorliegenden Erfindung;
- Fig. 3a-10e: Transport- und Verpackungsbehälter gemäß einer weiteren Ausführungsformen zur Verwendung bei einem Verfahren gemäß der vorliegenden Erfindung;
- Fig. 11a und 11b: eine weitere Variante für eine Prozessstation zum Bördeln eines Metalldeckels auf einem oberen Rand einer Mehrzahl von Behältern bei einem Verfahren gemäß der vorliegenden Erfindung;
- Fig. 12a - 12d: eine weitere Variante für eine Prozessstation bei einem Verfahren gemäß der vorliegenden Erfindung;
- Fig. 13a - 13e: Einzelheiten einer weiteren Haltestruktur gemäß einer weiteren Ausführungsform zur Verwendung bei einem Verfahren gemäß der vorliegenden Erfindung; und
- Fig. 14a - 14i: Einzelheiten einer weiteren Haltestruktur gemäß einer weiteren Ausführungsform zur Verwendung bei einem Verfahren gemäß der vorliegenden Erfindung.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

Eine Haltestruktur (Träger, im Stand der Technik häufig auch als sog. "Nest" bezeichnet) sowie ein Transport- und Verpackungsbehälter (im Stand der Technik häufig auch als "Tub" bezeichnet), der eine solche Haltestruktur aufnimmt, dienen gemäß der vorliegenden Erfmdung, wie nachfolgend beschrieben, der gleichzeitigen Halterung einer Mehrzahl von Behältern zur Aufbewahrung von Substanzen für kosmetische, medizinische oder pharmazeutische Anwendungen in einer regelmäßigen Anordnung, insbesondere in einer Matrixanordnung unter regelmäßigen Abständen der Behälter zueinander, entlang von zwei unterschiedlichen Raumrichtungen, bevorzugt entlang von zwei zueinander orthogonalen Raumrichtungen oder in regelmäßigen Reihen, die relativ zueinander versetzt angeordnet sind.

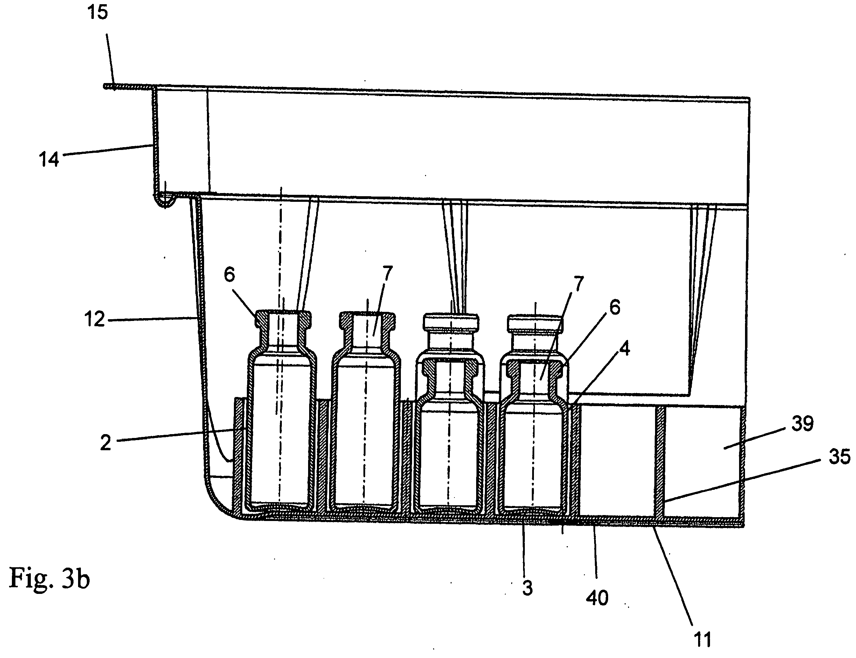

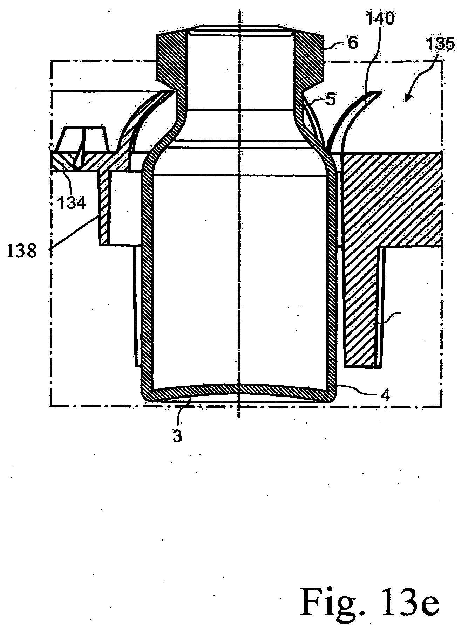

Ein Beispiel für derartige Medikamentenbehälter in Gestalt von Fläschchen (Englisch: vial) ist in der Fig. 3b oder Fig. 13e schematisch in einem Längsschnitt dargestellt und diese weisen eine zylindrische Grundform auf, mit einer zylinderförmigen Seitenwand mit - im Rahmen der Toleranzen - konstantem Innen- und Außendurchmesser, die von einem flach ausgebildeten Flaschenboden 3 senkrecht abragt und nahe dem oberen offenen Ende des Fläschchens in einen verengten Halsabschnitt 5 von vergleichsweise geringer axialer Länge und anschließend in einen verbreiterten oberen Rand 6 übergeht, der einen größeren Außendurchmesser aufweist als der zugeordnete Halsabschnitt 5 und zur Verbindung mit einem Verschlusselement ausgelegt ist. Der Halsabschnitt 5 kann glattwandig ohne Außengewinde ausgebildet sein oder kann mit einem Außengewinde zum Aufschrauben eines Verschlusselements versehen sein. Beispielsweise kann in die Innenbohrung des Halsabschnitts 5 und des oberen Rands 6 ein Stopfen (nicht dargestellt) eingeführt werden, dessen oberes Ende mit dem oberen Rand 6 des Fläschchens gasdicht und geschützt gegen das Eindringen von Verunreinigungen in das Fläschchen mit dem oberen Rand 6 verbunden ist, beispielsweise durch Bördeln einer nicht dargestellten Metallschutzfolie. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Behälter im Sinne der vorliegenden Anmeldung sind Ampullen, Karpullen oder Spritzen- oder Injektionsbehältnisse. Ampullen oder Karpullen sind Behältnisse für Arzneimittel zur meist parenteralen Applikation (Injektion), für Kosmetika und andere Substanzen und sind meist zylindrisch geformt, mit einer ausgezogenen Spitze (Spieß oder Kopf) und einem flachen Boden oder auch mit zwei ausgezogenen Spitzen an beiden Enden. Diese können insbesondere als Brechampullen mit einer ringförmigen Sollbruchstelle um den Ampullenhals herum oder als OPC-Ampulle (One-Point-Cut-Ampulle) mit einem in das Glas geritzten Brechring ausgebildet sein. Spritzen- bzw. Injektionsbehältnisse, auch als Injektionsfläschchen, Stechampulle oder Mehrwegampulle bezeichnet, sind zylindrische, flaschenähnlich geformte Behältnisse aus Glas oder Kunststoff, meist in relativ kleinen Nennvolumina (z. B. 1 ml, 10 ml). Sie sind mit einem Gummistopfen mit Septum (Durchstichgummi) verschlossen. Zum Schutz des Septums und Fixierung des Gummistopfens ist noch ein äußerer Verschluss (Bördelkappe oder Krampe), oft aus Aluminiumblech, aufgebracht. Bei einer Karpule befindet sich die Flüssigkeit in einem Zylinder, der am einen Ende mit einem dicken Gummi- oder Kunststoffstopfen verschlossen ist. Dieser fungiert als Kolben, wenn der Inhalt mit einer Karpulenspritze ausgepresst wird. Am anderen Ende ist der Zylinder nur mit einer dünnen Membran verschlossen, die bei der Anwendung vom hinteren Ende der Karpulenkanüle (eine beidseitig angeschliffene Kanüle) durchstochen wird. Zylinderampullen werden häufig in der Zahnmedizin zur Lokalanästhesie verwendet. Spezielle Zylinderampullen mit besonders gestaltetem Vorderteil (z. B. Gewinde) werden zur Insulintherapie in Insulinpens verwendet.

Im Sinne der vorliegenden Erfindung dienen derartige Behälter (container) zur Aufbewahrung von Substanzen oder Wirkstoffen für kosmetische, medizinische oder pharmazeutische Anwendungen, die in einer oder auch mehrere Komponenten in fester oder flüssiger Form in dem Behälter aufbewahrt werden sollen. Gerade bei Glasbehältern können Aufbewahrungsdauern viele Jahre betragen, was insbesondere von der hydrolytischen Resistenz der verwendeten Glassorte abhängt. Während nachfolgend Behälter offenbart werden, die zylindrisch sind, sei darauf hingewiesen, dass die Behälter im Sinne der vorliegenden Erfindung auch ein anderes Profil haben können, beispielsweise ein quadratisches, rechteckförmiges oder vieleckiges Profil.

Unweigerlich weisen solche Behälter herstellungsbedingt Toleranzen auf, die gerade bei Glasbehältern einen oder mehrere Zehntel Millimeter betragen können. Um solche Fertigungstoleranzen kompensieren zu können und gleichzeitig zu gewährleisten, dass sämtliche Flaschenböden 3 in einer Ebene angeordnet werden können, werden die Glasbehälter erfindungsgemäß mittels eines Formschlusses oder eines Reibschlusses an einer Halterungsstruktur bzw. Träger fixiert. Dieser Form- oder Reibschluss kann im Bereich des verengten Halsabschnitts 5 der Behälter, an dem zylindrischen Seitenwandabschnitt 4 oder im Bereich des unteren Endes der Behälter 2, insbesondere am Boden 3 der Behälter 2 realisiert werden.

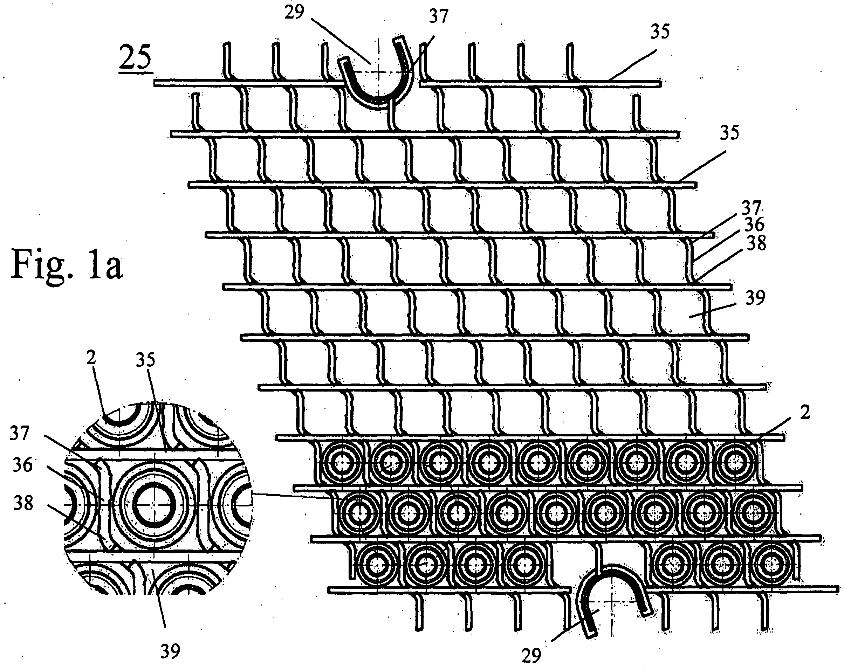

Die Fig. 1a zeigt eine Haltestruktur (Träger) 25 gemäß einem ersten Ausführungsbeispiel, mit reibschlüssiger Halterung der Behälter. Die Haltestruktur weist eine Mehrzahl von parallel zueinander verlaufenden Querstegen 35 auf, die über unter regelmäßigen Abständen zueinander angeordnete S-förmige Verbindungsstege 36 miteinander verbunden sind, die im Wesentlichen rechtwinklig zu den Querstegen 35 verlaufen. Genauer gesagt sind die Verbindungsstege 36 über in entgegengesetzte Richtungen gekrümmte vordere bzw. hintere Enden 37, 38 mit den Querstegen 35 verbunden. Die Verbindungsstege 36 sind aus einem Kunststoff hergestellt, bevorzugt aus einem flexiblen Kunststoff. Die Querstege 35 weisen bevorzugt eine größere Steifigkeit als die Verbindungsstege 36 auf. Aufgrund der S-förmigen Formgebung der Verbindungsstege 36 sind die Querstege 35 jeweils zueinander in deren Längsrichtung um eine konstante Distanz versetzt, so dass die Haltestruktur 35 insgesamt als Parallelogramm ausgebildet ist, mit einer Basis im Bereich des unteren Rands der in der Fig. 1a dargestellten Haltestruktur 25 und zwei dazu unter einem spitzen Winkel geneigt verlaufenden imaginären Randlinien, welche die vorderen Enden der Querstege 35 miteinander verbinden. In der im rechten Bildteil der Fig. 1a dargestellten entspannten Ausgangsposition können die Behälter 2 frei und ohne Berührung mit den Stegen 35, 36 oder jedenfalls mit nur geringem Kraftaufwand in die von den Stegen 35, 36 ausgebildeten länglichen Halteaufnahmen 39 eingeführt werden. Die Halteaufnahmen 39 weisen im Wesentlichen einen quadratischen Querschnitt auf, der so auf den Durchmesser der Behälter 2 abgestimmt ist, dass diese darin in einer zweiten Stellung der Haltestruktur 25 mit ausreichender Kraft reibschlüssig fixiert, insbesondere geklemmt werden können.

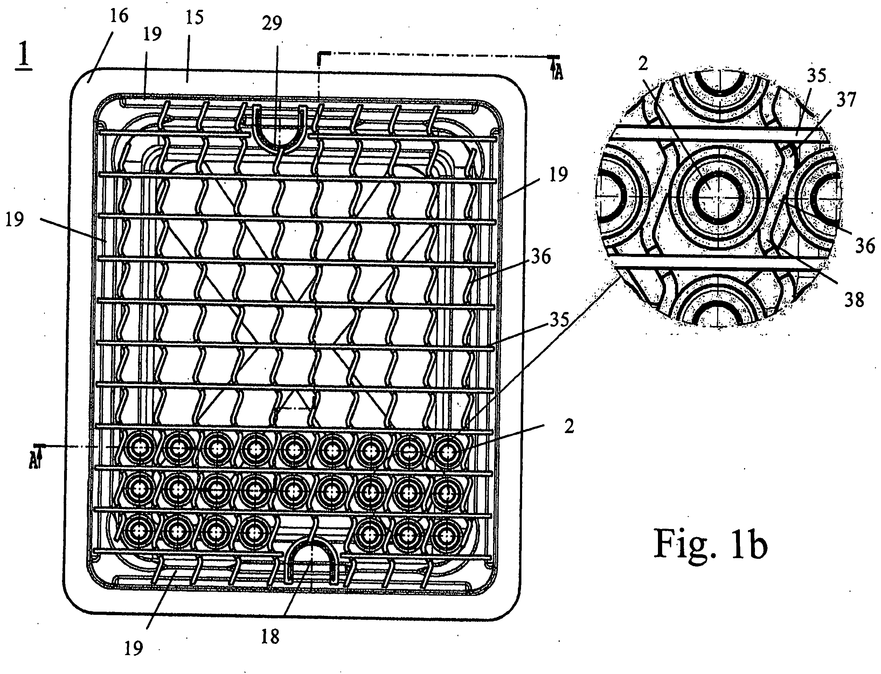

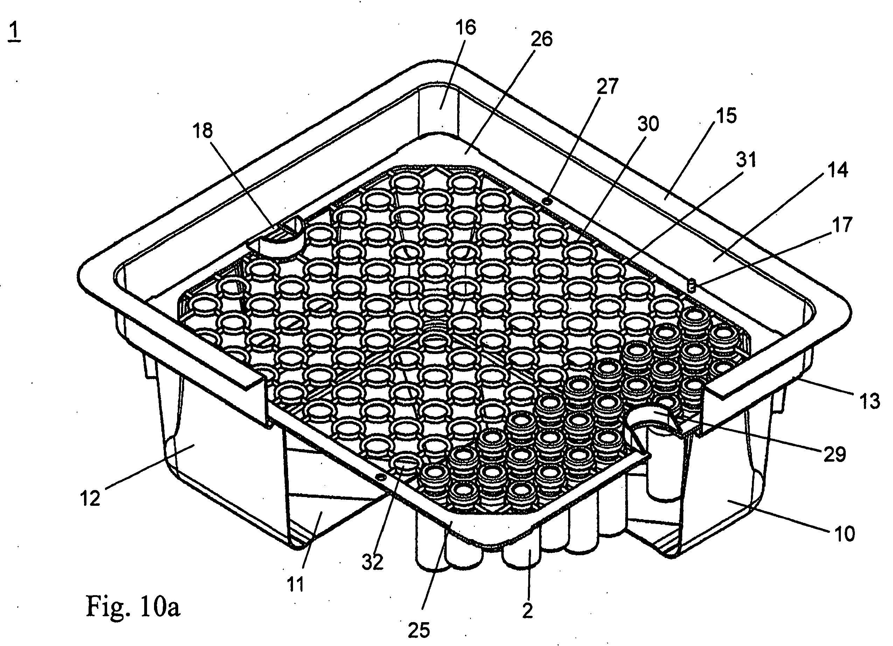

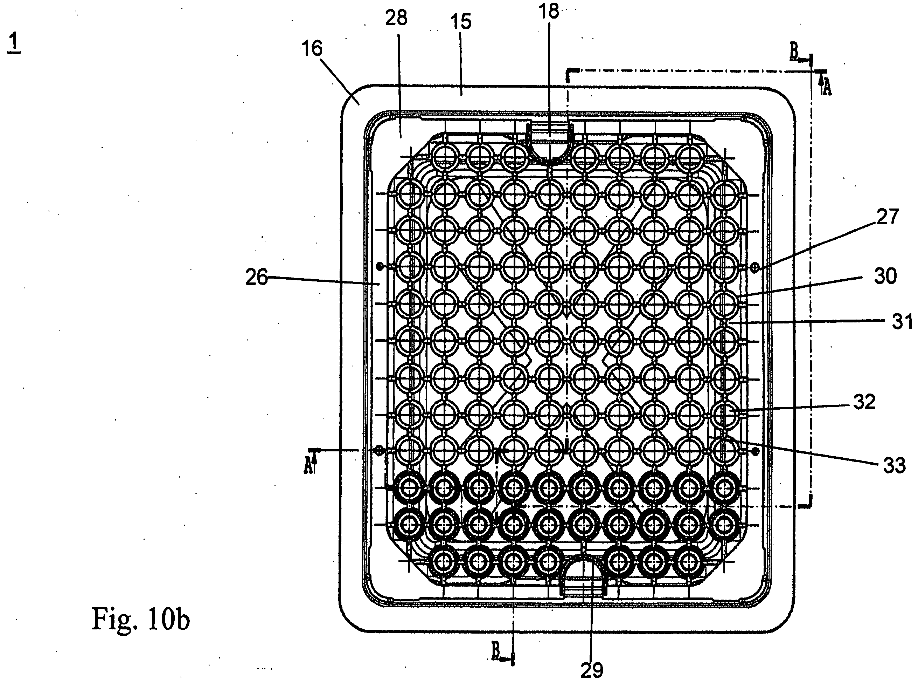

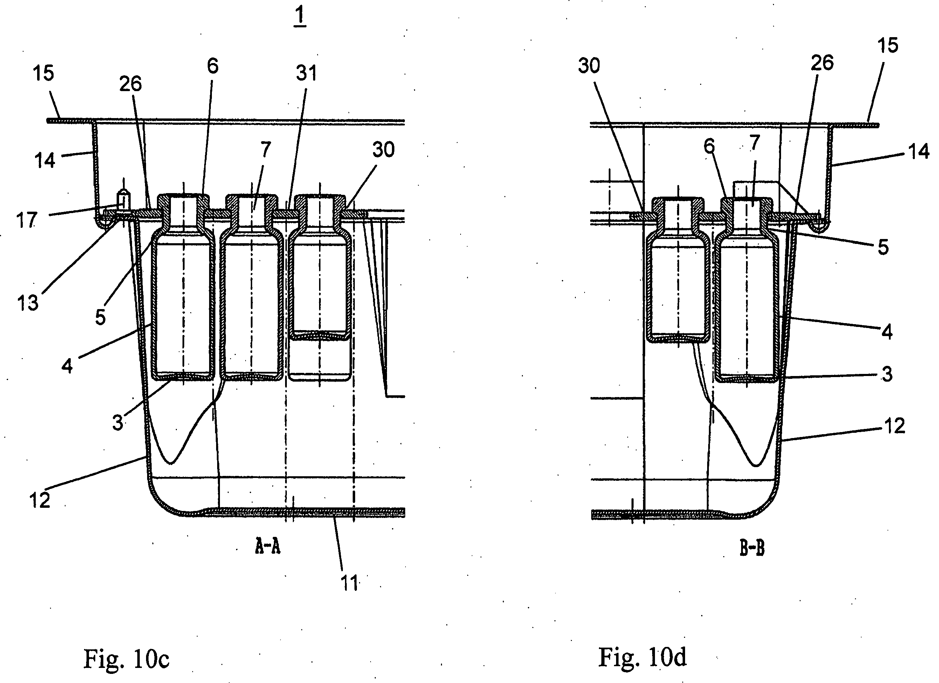

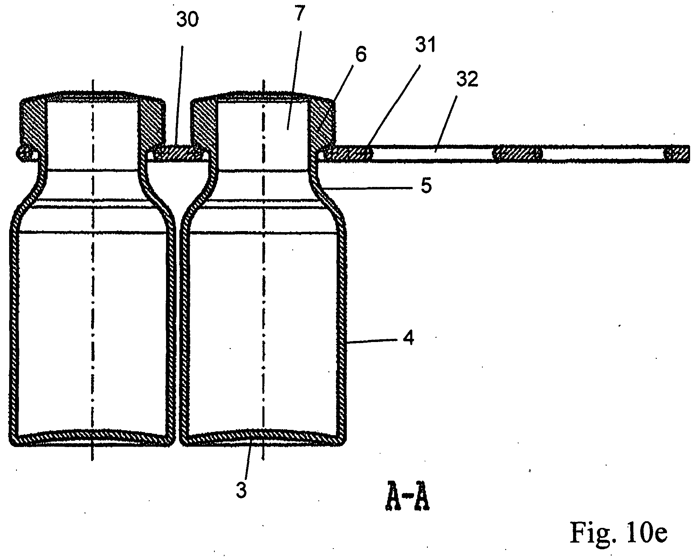

Um die Haltestruktur 25 aus der in der Fig. 1a dargestellten ersten Stellung in die in der Fig. 1b dargestellte zweite Stellung zu überführen, werden die Querstege 35 jeweils in deren Längsrichtung so verschoben, dass schließlich die in der Fig. 1b dargestellte rechteckförmige oder quadratische Haltestruktur 25 ausgebildet ist. Wie dem Vergleich der Figuren 1a und 1b entnommen werden kann, verbiegen sich hierzu die Verbindungsstege 36 geringfügig. Die zweite Stellung gemäß der Fig. 1b kann durch das Zusammenwirken der in der Haltestruktur 25 ausgebildeten Zugriffsöffnungen 29 mit korrespondierend ausgebildeten Gegenelementen des Behälters 10 fixiert werden oder auch durch das Zusammenwirken von Zentrieröffnungen 27 (vgl. Fig. 10a) mit korrespondierend ausgebildeten Zentrierzapfen 17 eines nicht dargestellten Halterahmens, welcher die Haltestruktur 25 aufnimmt.

Der vergrößerten Teildarstellung im unteren linken Teil der Fig. 1a kann man entnehmen, dass die Behälter 2 in der ersten Stellung gemäß der Fig. 1a lose in den Halteaufnahmen 39 aufgenommen sind. Der vergrößerten Teildarstellung im rechten oberen Teil der Fig. 1b kann man entnehmen, dass die Behälter 2 in der zweiten Stellung gemäß der Fig. 1b von zentralen Abschnitten der Verbindungsstege 36 reibschlüssig fixiert, insbesondere geklemmt, werden. Zu den Querstegen 35 kann weiterhin ein gewisses Spiel bestehen, das jedoch bevorzugt minimal oder verschwindend ist.

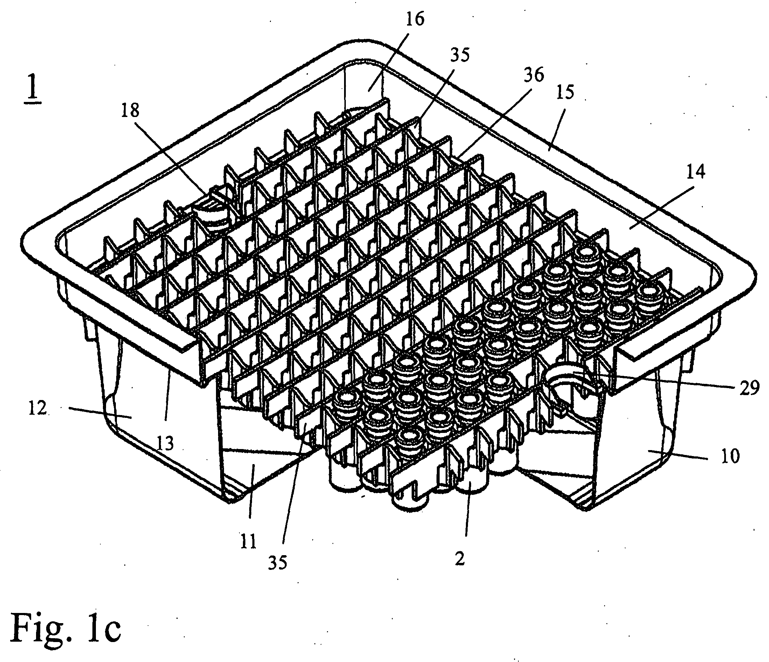

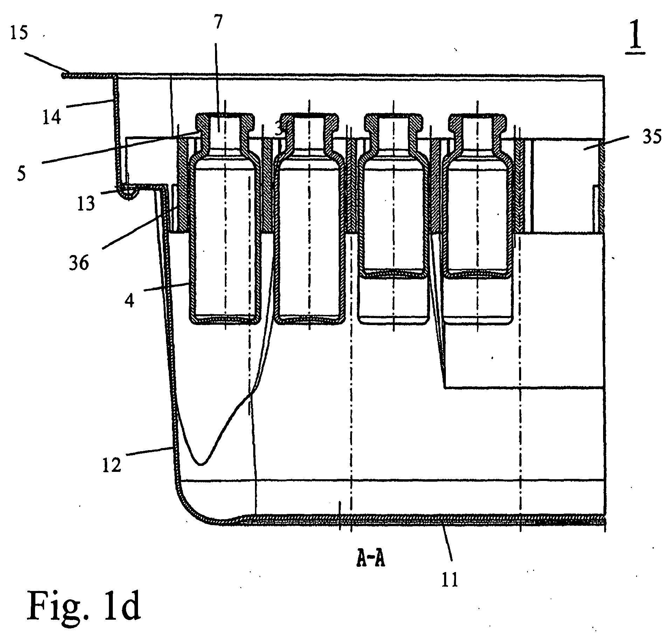

Die Fig. 1c zeigt die so ausgebildete Verpackungseinheit 1 in einem perspektivischen Teilschnitt, wobei erkennbar ist, dass bei dieser Ausführungsform die Querstege 35 unmittelbar auf der von der Stufe 13 des Behälters 10 ausgebildeten Abstützfläche abgestützt sind. Die reibschlüssige Fixierung der Behälter ist in dem Teilschnitt gemäß der Fig. 1d erkennbar.

Wie vorstehend ausgeführt, werden bei der zweiten Ausführungsform sämtliche Seitenwände der Aufnahmen 39 beim Verschieben der Querstege 35 koordiniert von der ersten Stellung in die zweite Stellung verstellt, und zwar durch Verschwenken des oberen Endes der Haltestruktur 25 (vgl. Fig. 1a) relativ zu der Basis am unteren Ende des in der Fig. 1a dargestellten Parallelogramms. In der vorgenannten ersten Stellung können die Behälter somit in die Haltestruktur eingeführt und präzise positioniert werden, insbesondere auf einem vorbestimmten Höhenniveau so angeordnet werden, dass sämtliche Böden der Behälter bündig und in einer gemeinsamen Ebene angeordnet sind. Durch koordiniertes Verstellen der Haltestruktur in die vorgenannte zweite Stellung werden dann sämtliche Behälter gleichzeitig reibschlüssig fixiert und präzise in einer regelmäßigen Anordnung positioniert. Die Haltestruktur gemäß den Figuren 1a bis 1c ist bevorzugt einstückig aus einem Kunststoff ausgebildet. Eine ähnlich wirkende Haltestruktur kann jedoch auch aus einer Mehrzahl identischer Grundeinheiten zusammengesteckt werden, wie nachfolgend beschrieben.

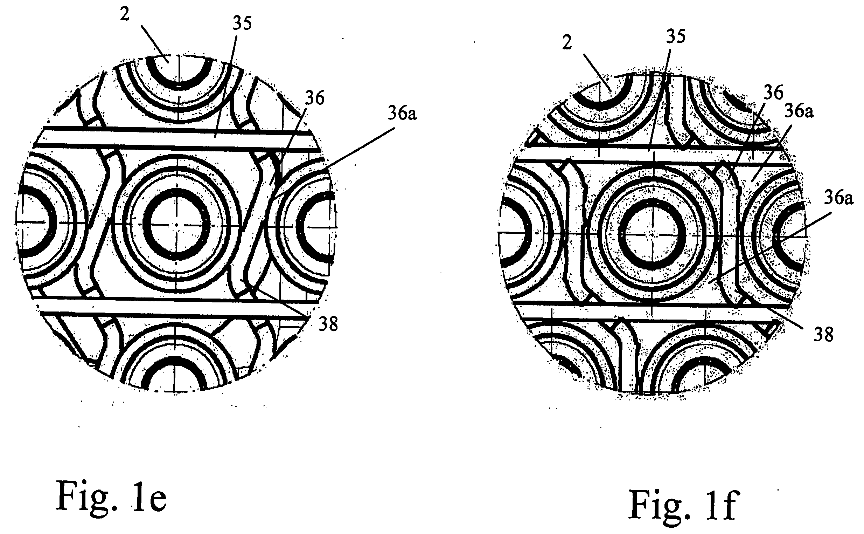

Die Figuren 1e und 1f zeigen in stark vergrößerten Teildarstellungen eine weitere Variante der Haltestruktur gemäß den Figuren 1a-1c. Dabei zeigt die Fig. 1e den Bereich einer Aufnahme der Haltestruktur in der vorgenannten zweiten Stellung, in der die Behälter 2 in den Aufnahmen der Haltestruktur gehalten sind. Abweichend zu den Figuren 1a-1c ist bei dieser Ausführungsform an den Verbindungsstegen 36 auf den beiden Seiten jeweils ein konkav ausgebildeter Abschnitt 36a ausgebildet, wobei der Krümmungsradius der beiden konkaven Aufnahmen der Abschnitte 36a auf den Radius der Behälter 2 abgestimmt ist. In der zweiten Stellung gemäß der Fig. 1e, in welcher die Verbindungsstege 36 sich geneigt zu den Querstegen 35 erstrecken, schmiegen sich die konkaven Aufnahmen 36a an die zylindrische Seitenwand der Behälter 2 an, sodass die Behälter noch zuverlässiger und kontrollierter gehalten werden können. In der ersten Stellung gemäß der Fig. 1f, in welcher die Verbindungsstege 36 sich senkrecht zu den Querstegen 35 erstrecken, sind die konkaven Aufnahmen 36a nicht mehr gegenüberliegend zur zylindrischen Seitenwand der Behälter 2 angeordnet, sodass die Behälter ungehindert, jedenfalls mit deutlich reduziertem Kraftaufwand, in die von den Stegen 35, 36 ausgebildeten Aufnahmen eingeführt und aus diesen herausgenommen werden können. Idealerweise liegen die Stege 35, 36 in der ersten Stellung gemäß der Fig. 1f überhaupt nicht an den Seitenwänden der Behälter 2 an.

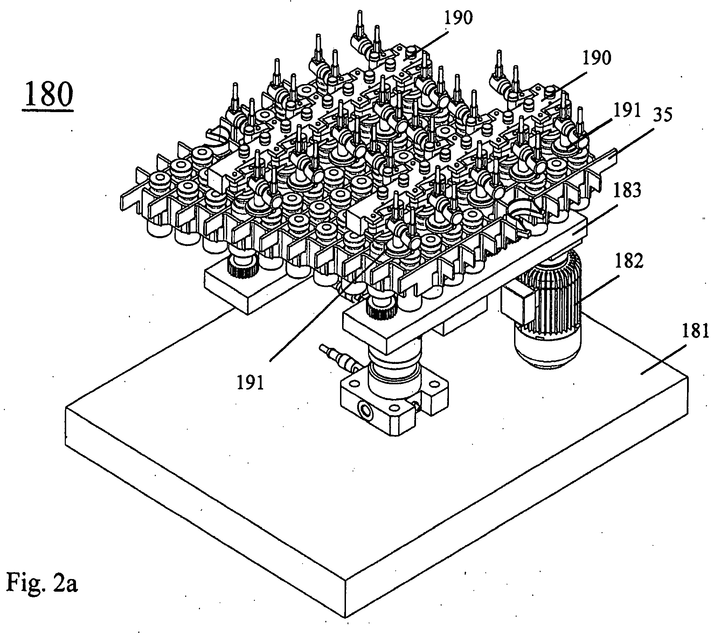

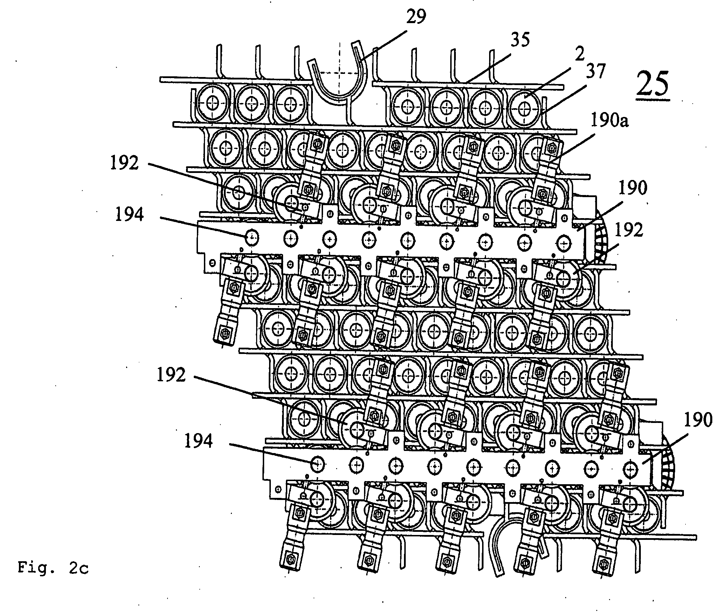

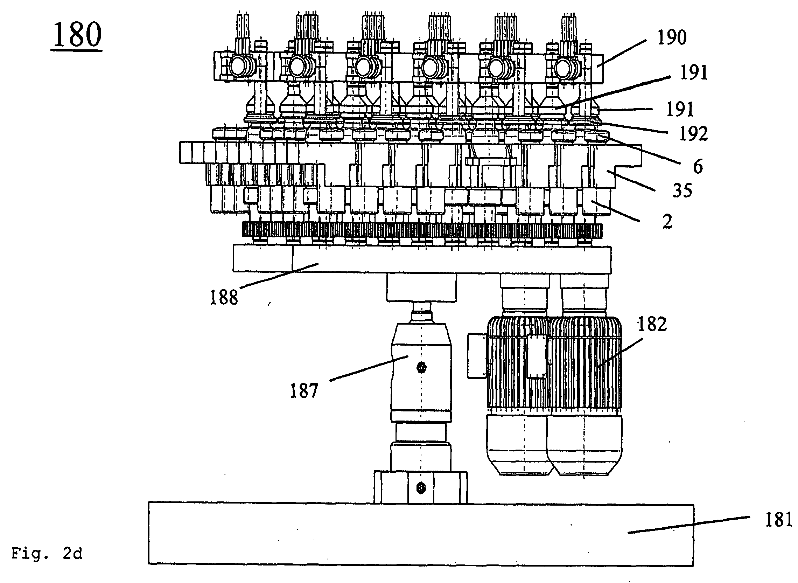

Die Figuren 2a bis 2d zeigen als ein Beispiel für eine Prozessstation zur Behandlung oder Verarbeitung von Medikamentenbehältern eine Bördelstation 180 zum gleichzeitigen Bördeln von Metalldeckeln auf den oberen Rändern von Behältern 2, die in einem Träger, wie vorstehend anhand der Figuren 1a bis 1c beschrieben, aufgenommen oder gehalten werden. Nicht dargestellt ist hier die Fördereinrichtung, beispielsweise ein Transportband oder eine Rollenstraße in einem Steriltunnel. Die Bördelstation 180 ist auf einem Gestellrahmen 181 angeordnet, der sich unterhalb der Fördereinrichtung befindet und im Regelfall von der Prozessanlage möglichst getrennt ist. Die Bördelstation 180 weist einen Haltearm 183 oder eine Abdeckung auf, der bzw. die von Hubstangen 185 durchgriffen wird, an deren oberem Ende jeweils ein Drehteller 186 drehbeweglich gelagert ist, der von dem zugeordneten Drehantrieb 182 und über das Getriebe 184 gedreht werden kann.

Oberhalb der Behälter befindet sich eine Halterung 190, an der eine Mehrzahl von Zentrierscheiben 191 und Bördelscheiben 192 gehalten sind, deren Position präzise eingestellt werden kann, in Anpassung an die regelmäßige Anordnung der zu verarbeitenden Behälter, deren Anordnung durch die Querstege 35 und Aufnahmen des Trägers festgelegt wird.

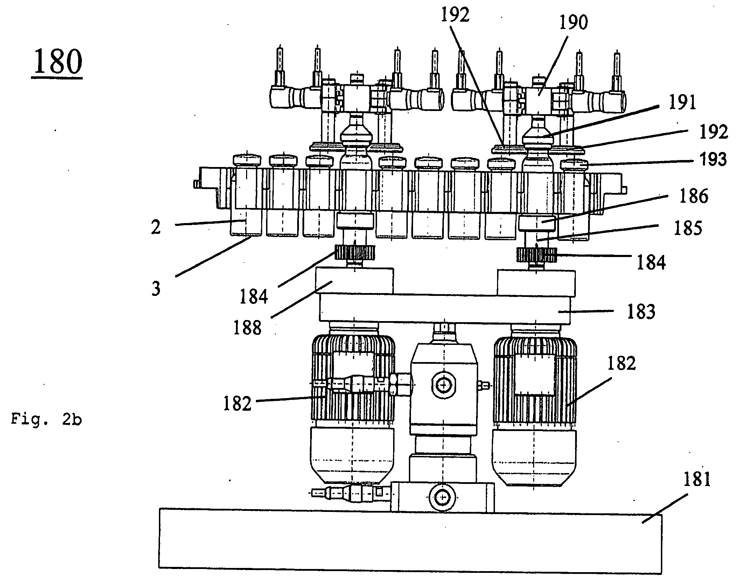

Während die Träger in der Konfiguration gemäß der Fig. 1b stromaufwärts der Prozessstation gefördert werden, also beispielsweise in einem Transport- und Verpackungsbehälter gemäß der Fig. 1c mit reibschlüssiger Halterung der Behälter 2, wird der Träger aus dem Transport- und Verpackungsbehälter entnommen und in die Stellung gemäß der Fig. 1a überführt, in welcher der Reibschluss zwischen den Querstegen 35 und den Behältern 2 größtenteils oder vollständig aufgehoben ist. Damit die Behälter nicht aus den Aufnahmen des Trägers nach unten unkontrolliert herausrutschen, sind in dem Bereich der Bördelstation 180 geeignete Abstütz- und Führungsflächen (nicht dargestellt) vorgesehen. Alternativ werden die Behälter 2 in der Stellung des Trägers gemäß der Fig. 1a nur noch mit geringer Kraft geklemmt, sodass diese in den Aufnahmen des Trägers ohne weiteres durch Hochfahren der Hubstangen 185 in eine angehobene Position hochgeschoben werden können. In dieser angehobenen Stellung sind die oberen Ränder der Behälter 2 für das Bördeln in ausreichendem Maß frei zugänglich. Wie man dem Querschnitt gemäß der Fig. 2b entnehmen kann, werden nur einzelne der Behälter 2 mittels der Hubstangen 185 hochgeschoben. In der angehobenen Stellung ist der Reibschluss zwischen den Querstegen 35 des Trägers und den Behältern 2 bevorzugt vollständig aufgehoben.

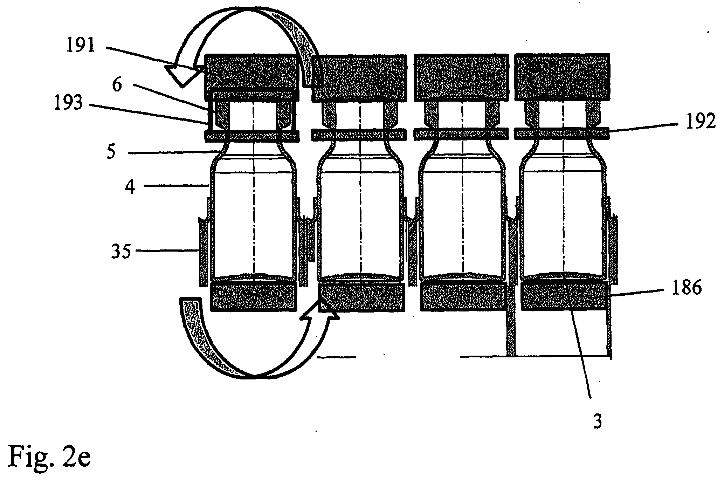

Wie man der vergrößerten Darstellung gemäß der Fig. 2e entnehmen kann, ist in der angehobenen Stellung der obere Rand der Behälter 2 mit dem darin eingesetzten Stopfen und einem Metalldeckel 193, beispielsweise einer Aluminiumkappe, in einer zugeordneten Zentrierscheibe 191 aufgenommen, um die Drehbewegung des Behälters beim Drehen des Drehtellers 186 zu zentrieren. Beim Drehen des Behälters 2 gelangt eine an dem Arm 190 gelagerte Bördelscheibe 192 in Anlage mit dem Metalldeckel 193 und bördelt diesen durch Umformung geeignet um, um so den Behälter steril zu versiegeln.

Wenn sämtliche Behälter 2 eines Trägers in der vorstehend beschriebenen Weise verarbeitet worden sind, wird der Träger aus dem Bereich der Bördelstation 180 abtransportiert und in der Prozessanlage (nicht dargestellt) weiter gefördert. Hierzu können die Behälter in den Öffnungen oder Aufnahmen des Trägers erneut in deren Normalstellung zurückgeschoben und die Träger erneut in die Stellung gemäß der Fig. 1b überführt werden, beispielsweise durch Einsetzen in einen Transport- und Verpackungsbehälter, um weiter gefördert zu werden. Die Verarbeitung in der Bördelstation erfolgt somit chargenweise, ohne dass die Behälter jedenfalls vollständig aus dem Träger entnommen zu werden brauchen. Die Querstege 35 des Trägers verhindern zu jedem Zeitpunkt der Verarbeitung eine Kollision von unmittelbar benachbarten Behältern, was einen unerwünschten Abrieb und eine Verunreinigung der Prozessanlage verhindert.

Mit Hilfe eines Trägers gemäß der Fig. 1a oder wie nachfolgend beschrieben, können somit erfindungsgemäß eine Mehrzahl von Behältern gleichzeitig oder chargenweise behandelt oder verarbeitet werden, während diese an einem Träger gehalten oder jedenfalls geführt sind. Gemäß weiteren Ausführungsformen der Erfindung kann eine solche Behandlung oder Verarbeitung auch dann erfolgen, während der Träger mit den von diesem gehaltenen Behältern in einem Transport- und Verpackungsbehälter angeordnet ist, wie dieser beispielhaft in der Fig. 1c dargestellt ist. Hierzu kann der Träger 25 (vgl. Fig. 1c) beispielsweise über die Zugriffsöffnungen 29 geeignet angehoben werden, ohne jedoch aus dem Transport- und Verpackungsbehälter 1 entnommen zu werden. Geht trotz aller Vorsichtsmaßnahmen einmal ein Behälter zu Bruch oder kommt es in anderer Weise während der Behandlung oder Verarbeitung zu Verunreinigungen, so werden diese Verunreinigungen jedenfalls in dem Transport- und Verpackungsbehälter zurückgehalten, geraten somit jedenfalls nicht in die eigentliche Prozessanlage.

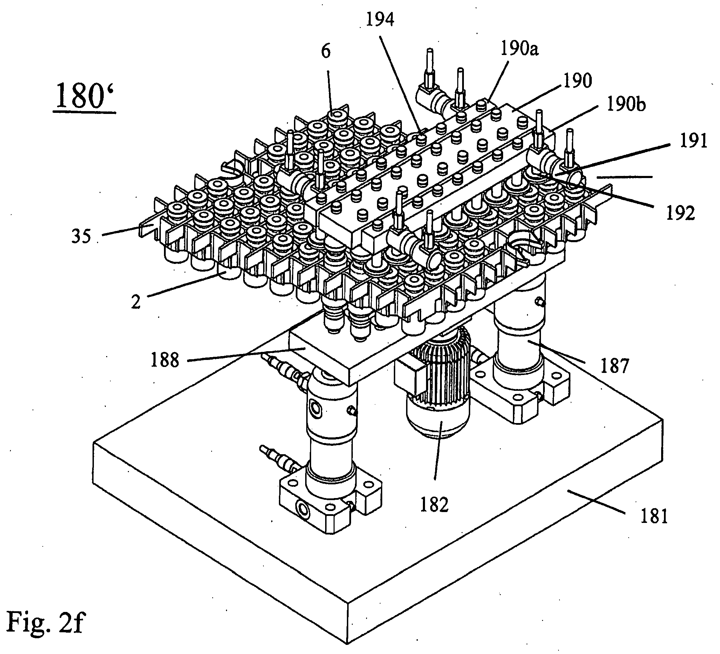

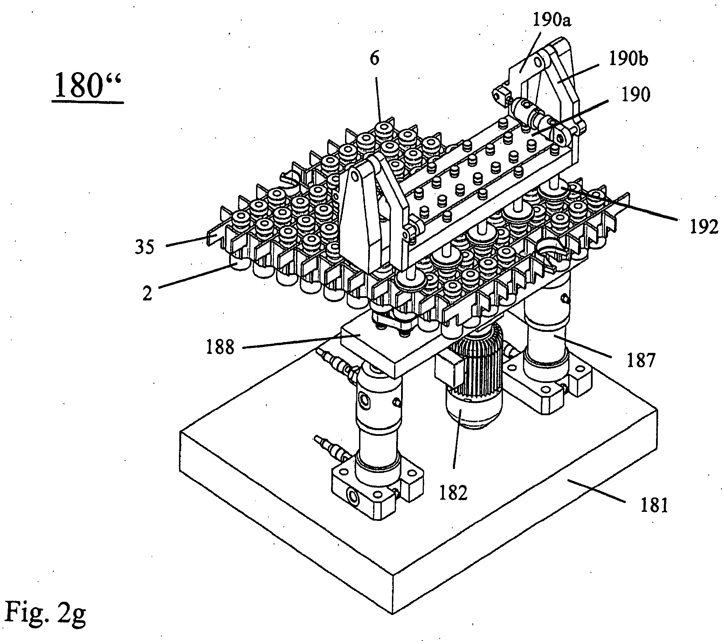

Die Figuren 2f und 2g zeigen weitere Varianten der Bördelstation gemäß der Fig. 2a. So sind bei der Bördelstation gemäß der Fig. 2f die Zentrierscheiben 191 an einem zentralen Haltearm 190 gelagert, während die Bördelscheiben 192 an sich parallel zu diesem erstreckenden Haltearmen 190a, 190b gelagert sind, deren Abstand und Ausrichtung relativ zu dem zentralen Haltearm 190 geeignet eingestellt werden kann, beispielsweise in Anpassung an den jeweils zu verarbeitenden Behältertyp mit unterschiedlichen Abmessungen. Bei der Variante gemäß der Fig. 2g erfolgt diese Verstellung durch geeignete Verstellung der schwenkbeweglich an dem zentralen Haltearm 190 gelagerten seitlichen Haltearme 190a, 190b.

Bei dem erfindungsgemäßen Verfahren befinden sich die Bördelschreiben stets nur über bereits mit Hilfe eines Stopfens verschlossenen Behältern. Ein Eindringen von Verunreinigungen oder Abrieb in den Behälterinnenraum kann also erfindungsgemäß zuverlässig ausgeschlossen werden.

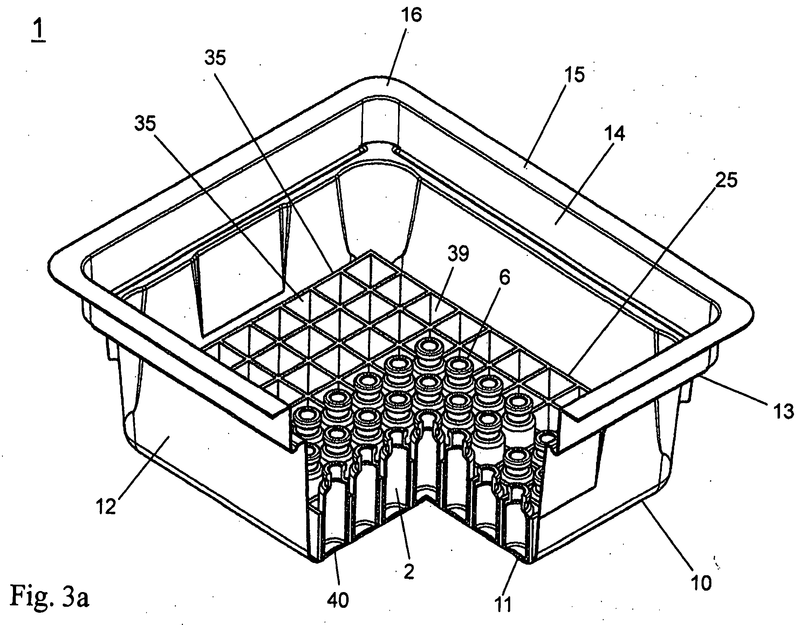

Während vorstehend dargelegt wurde, dass die Haltestruktur auf der von der Stufe 13 nahe dem oberen Rand ausgebildeten Auflagefläche abgestützt ist, ist nach dem Ausführüngsbeispiel gemäß der Fig. 3a die Haltestruktur 25 unmittelbar auf dem Boden 11 des Transport- und Verpackungsbehälters 10 abgestützt. Dies funktioniert grundsätzlich bei sämtlichen Haltestrukturen, die über eine gewisse axiale Länge verfügen, sei es dass die Böden der Behälter 2 unmittelbar auf dem Boden 11 des Behälters 10 aufliegen oder auf Böden der länglichen Aufnahmen 39 der Haltestruktur 25 aufliegen. Bei dem dargestellten Ausführungsbeispiel gemäß der Fig. 3a ist die Haltestruktur 25 durch eine Mehrzahl von sich rechtwinklig kreuzenden Querstegen 35 ausgebildet, die eine Mehrzahl von länglichen Aufnahmen 39 mit quadratischem Querschnitt ausbilden, wobei die Aufnahmen 39 in einer Matrix-Anordnung angeordnet sind. Dabei werden die Behälter 2 an ihrem unteren Ende reibschlüssig von den Querstegen 35 oder einer darauf vorgesehenen Einlage aus einem flexiblem Kunststoff fixiert. Die Fig. 3b zeigt einen schematischen Teilschnitt durch diese Haltestruktur. Dargestellt ist beispielhaft die Klemmung von Behältern 2 mit unterschiedlicher Höhe. Grundsätzlich kann die Haltestruktur 25 auch einstückig mit dem Boden 11 des Behälters 10 ausgebildet sein.

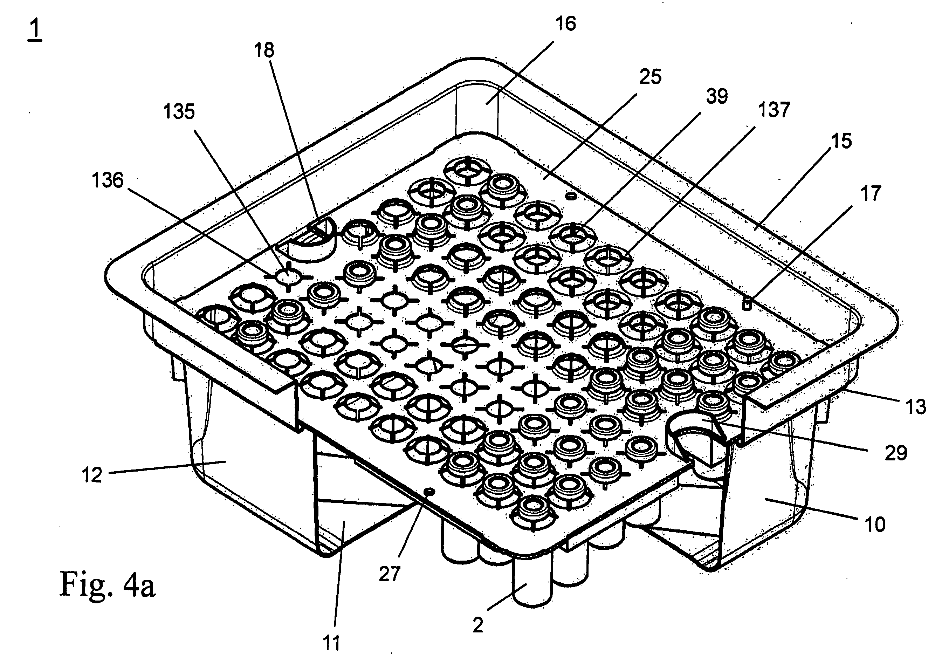

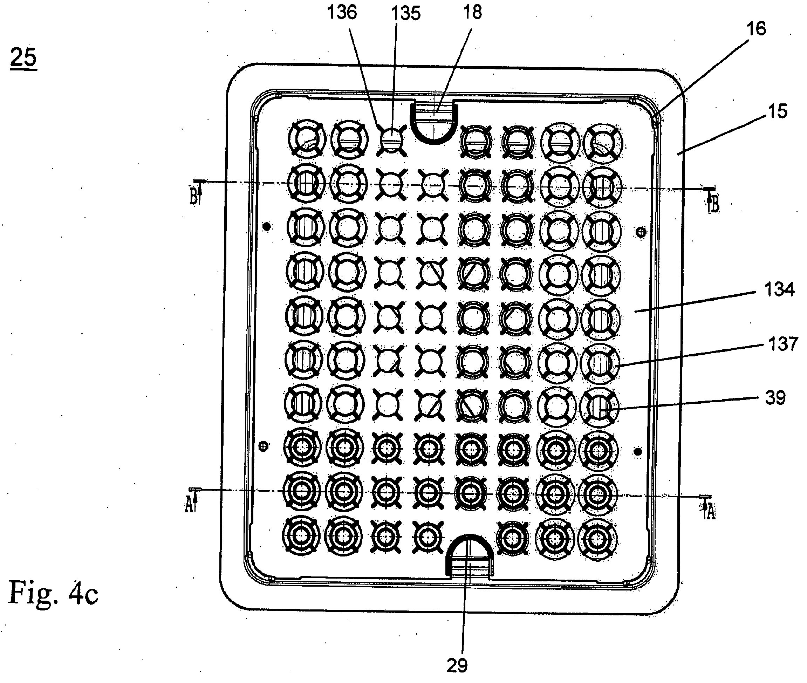

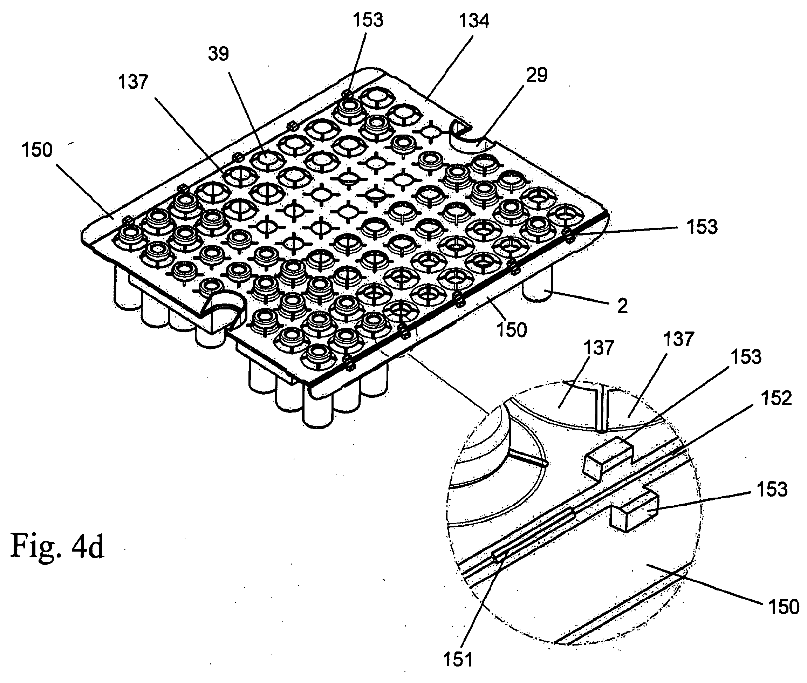

Die Figuren 4a bis 4c zeigen ein weiteres Ausführungsbeispiel für eine Haltestruktur (Träger), woran eine Mehrzahl von Behältern 2 formschlüssig gehalten werden können. Hier sind gemäß der Fig. 4a in der flächigen Transportplatte 25 eine Mehrzahl von Öffnungen 39 zur Aufnahme der Behälter 2 ausgebildet.

Die Öffnungen sind in ringförmigen Formschlusselementen 137 ausgebildet, die entweder in die Öffnungen 39 eingesteckt sind, insbesondere in deren Umfangsrand eingerastet oder eingeclipst sind, oder die einstückig mit dem flächigen Träger 25 ausgebildet sind, beispielsweise durch ein 1 K- oder 2K-Kunststoff-Spritzgussverfahren.

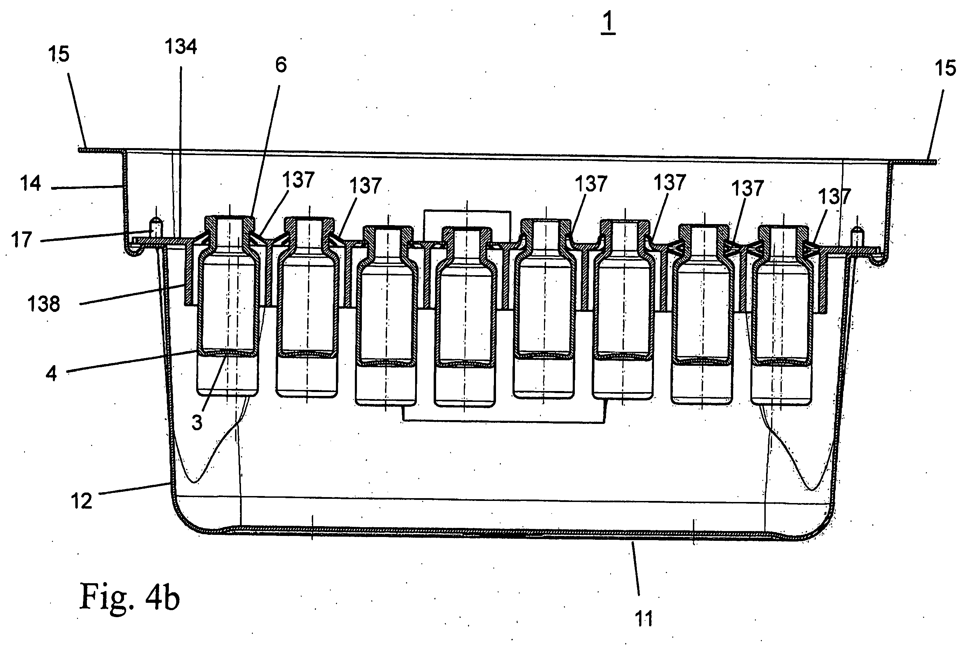

Der schematische Längsschnitt gemäß der Fig. 4b stellt summarisch mehrere verschiedene Ausführungsvarianten für Formschlusselemente 137 in einer vergleichenden Darstellung dar.

Im fixierten Zustand stützen die Formschlusselemente 137 die Behälter unmittelbar unterhalb des oberen Rands 6 und im Bereich des verengten Halsabschnitts 5 ab.

Wenngleich in der Fig. 4a der Boden 11 des Behälters 10 als geschlossen und einstückig mit der Seitenwand 12 ausgebildet dargestellt ist, kann das untere Ende des Behälters 10 auch in der Art des oberen Endes geöffnet ausgebildet sein, insbesondere mit einem flanschartigen unteren Rand in der Art des oberen Rands 15 versehen sein, so dass die Böden der Behälter 2 von der Unterseite des Behälters 10 her frei zugänglich sind, beispielsweise für Verarbeitungsschritte in einem Steriltunnel oder in einem Gefriertrockenschrank, wie nachfolgend näher erläutert.

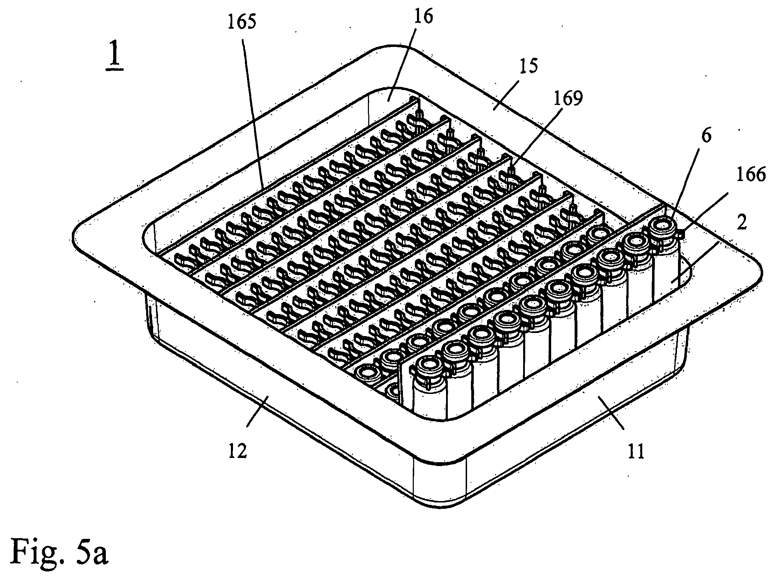

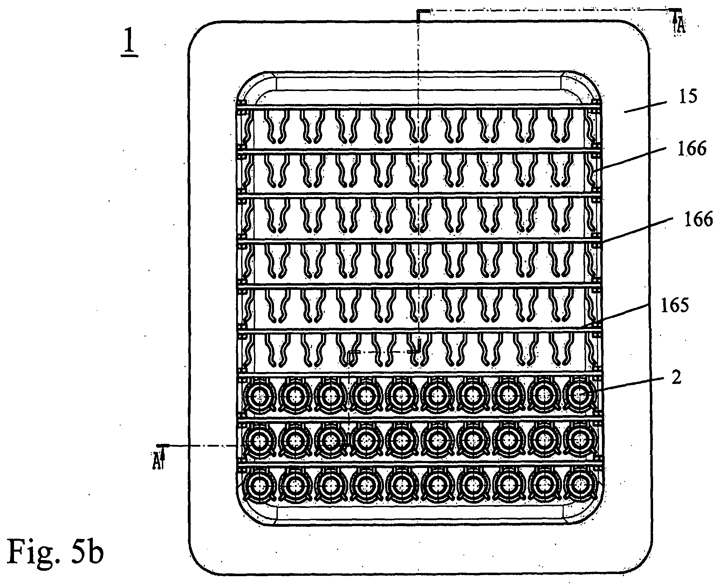

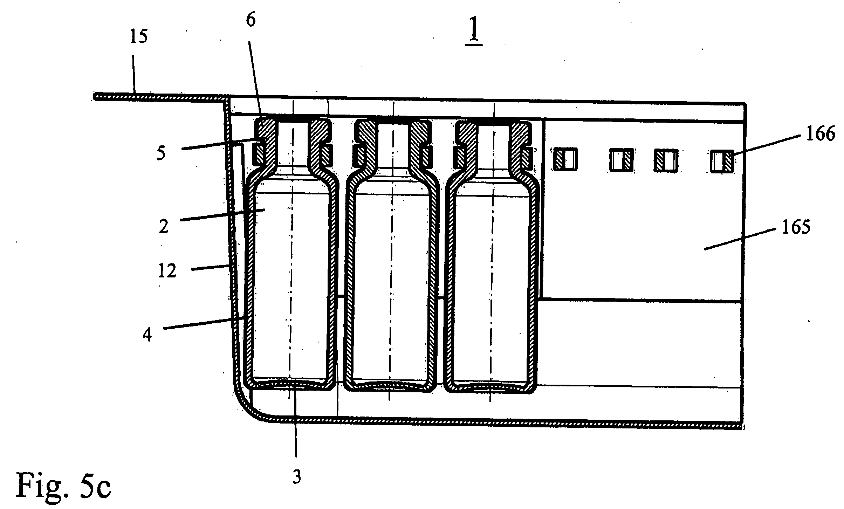

Die Figuren 5a bis 5c zeigen eine weitere Ausführungsform eines Transport- und Verpackungsbehälters 1. Gemäß der Fig. 5a ist die Haltestruktur 25 als Kasten ausgebildet, mit umlaufenden Seitenwänden 168. Dieser Kasten 168 wird von einer Mehrzahl von sich parallel erstreckenden und zueinander unter regelmäßigen Abständen zueinander beabstandeten Querstegen 165 in mehrere rechteckförmige Segmente unterteilt. Auf den Oberflächen der Querstege 165 sind auf gleicher Höhe und unter regelmäßigen Abständen zueinander konkav gebogene Haltearme 166 angeordnet, die aus einem elastischen Kunststoff ausgebildet sind und entweder einstückig mit den Querstegen 165 ausgebildet sind oder an diesen befestigt oder angeformt sind. Die Haltearme 166 bilden Aufnahmen, in welche die Behälter so von vorne her eingeführt werden können, dass deren Halsabschnitte formschlüssig umgriffen und deren obere Ränder auf diesen abgestützt werden können, wie in der Fig. 5c dargestellt. In Längsrichtung der Behälter 2 betrachtet besteht ein Reibschluss. Durch Aufbringen einer geeigneten axial wirkenden Kraft können die Behälter 2 jedoch in den von den Haltearmen 166 ausgebildeten Aufnahmen axial verschoben werden, beispielsweise in eine angehobene Stellung, wie vorstehend anhand der Figuren 2a bis 2d beschrieben.

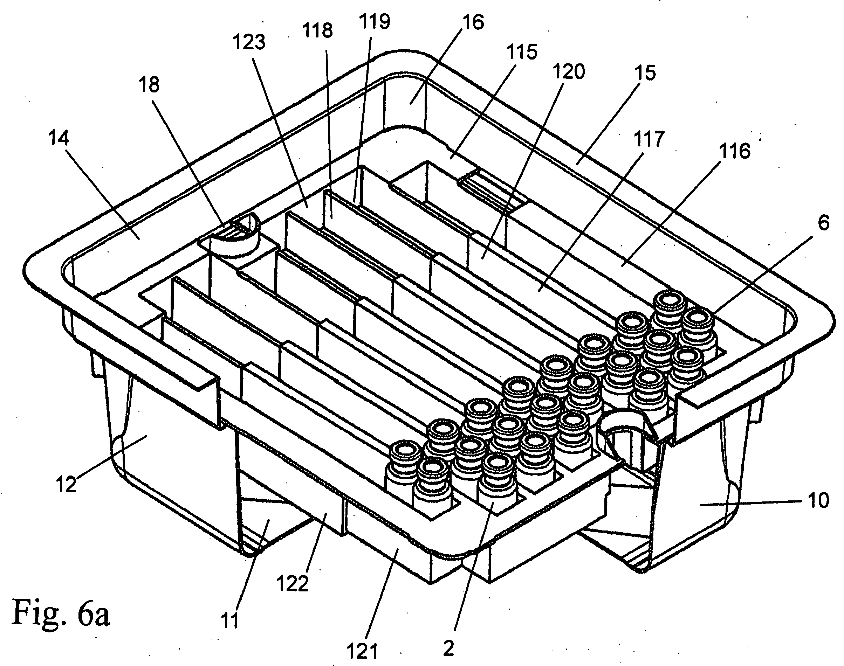

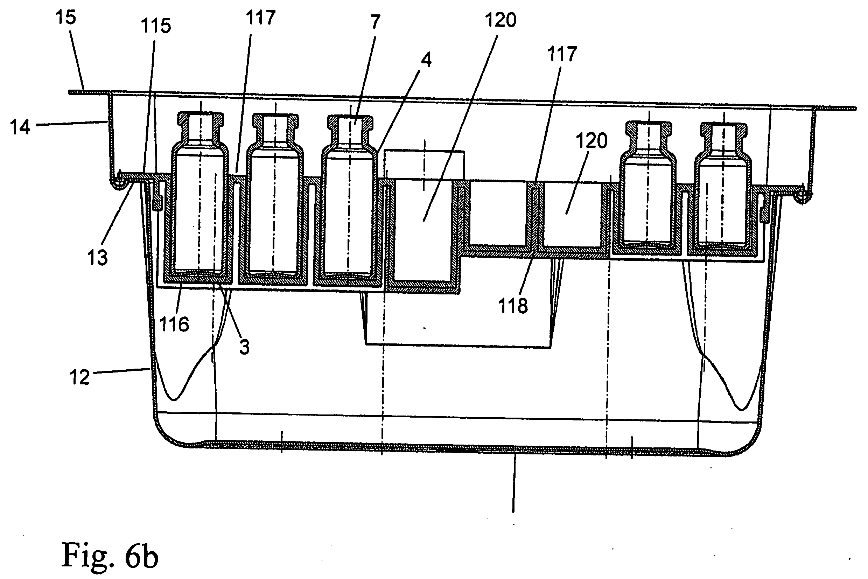

Die Fig. 6a zeigt eine weitere Ausführungsform für einen Transport- und Verpackungsbehälter 1 mit einem Halteeinsatz, der von zwei ineinander schiebbaren Teilen ausgebildet ist, die gemeinsam längliche, im Profil rechteckförmige und parallel zueinander verlaufende Halteaufnahmen 120 ausbilden, in welchen die Behälter 2 aufgenommen und reibschlüssig fixiert sind. Genauer gesagt sind in dem rechten Schiebeteil 116 von den Führungswänden 117 eine Mehrzahl von sich parallel zueinander erstreckenden rechteckförmigen Aufnahmen 120 ausgebildet und sind in dem linken Schiebeteil 115 von den Seitenwänden 118 entsprechende längliche Aufnahmen ausgebildet. Die Führungswände 117 sind geschlitzt ausgebildet, wie man der Schnittansicht gemäß der Fig. 6b entnehmen kann. In den Längsschlitzen der Führungswände 117 sind die korrespondierend ausgebildeten Seitenwände 118 des linken Schiebeteils 115 verschieblich geführt und aufgenommen. Die beiden Schiebeteile 115, 116 können ineinander geschoben werden, bis vordere bzw. hintere Enden 123 der Aufnahmen 120 unmittelbar an den in den Aufnahmen 120 aufgenommenen Behälter 2 anliegen, so dass die Behälter 2 von den Seitenwänden der Aufnahmen 120 gemeinsam geklemmt werden. Grundsätzlich können die Seitenwände der Aufnahmen 120 mit Haltevorsprüngen bzw. Vertiefungen korrespondierend zu den Behältern 2 ausgebildet sein, so dass die Behälter 2 einander nicht unmittelbar berühren, wenn diese in den Aufnahmen 120 aufgenommen sind, sondern in den von den Vorsprüngen bzw. Vertiefungen ausgebildeten Aufnahmen beabstandet zueinander aufgenommen sind.

Gemäß einer bevorzugten weiteren Ausführungsform sind die Seitenwände 118, 122 des linken Schiebeteils 115 in Längsrichtung der Aufnahmen 120 betrachtet keilförmig ausgebildet, so dass die Öffnungsweite zwischen den Seitenwänden 117 des rechten Schiebeteils 116 zunehmend verringert wird, solange bis die Behälter 2 in den länglichen Aufnahmen 120 reibschlüssig fixiert, insbesondere geklemmt sind. Die Fig. 6b ist eine schematische Schnittansicht und zeigt die Aufnahme von Behältern mit unterschiedlichen Höhen in Aufnahmen 120 mit unterschiedlichen Höhen (linker und rechter Bildteil).

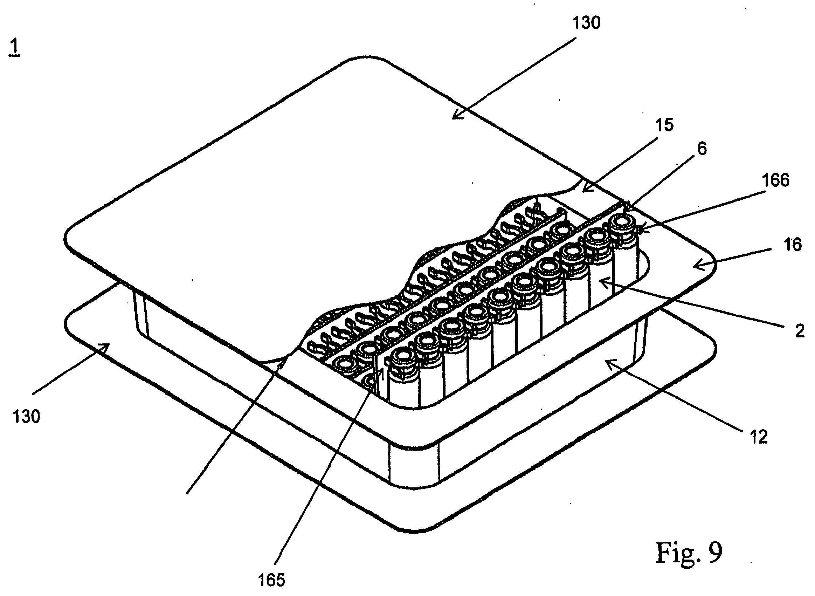

Die Oberseite oder die Ober- und Unterseite einer Haltestruktur 25 gemäß der vorliegenden Erfindung oder auch eines Transport- und Verpackungsbehälters 1 gemäß der vorliegenden Erfindung kann bzw. können von einer sterilen, gasdurchlässigen Schutzfolie überzogen sein, die aufgeklebt und bei Bedarf abziehbar ist. Dies ist beispielhaft in der Fig. 9 für eine Verpackungseinheit dargestellt, die von einem beidseitig offenen Transportbehälter und einer darin aufgenommen Haltestruktur gemäß der Fig. 5a ausgebildet ist und auf der Ober- und Unterseite mittels einer auf den Rand 15 aufgeklebten Schutz- oder oder Verpackungsfolie 130 verschlossen ist. Bei der Schutzfolie 130 kann es sich insbesondere um eine gasdurchlässige Kunststofffolie handeln, insbesondere um ein Geflecht aus Kunststofffasern, beispielsweise aus Polypropylen-Fasern (PP) oder auch um eine Tyvek®-Schutzfolie, die eine Sterilisation der in der Haltestruktur 25 aufgenommenen und verpackten Behälter 2 durch die Folie 130 hindurch ermöglicht.

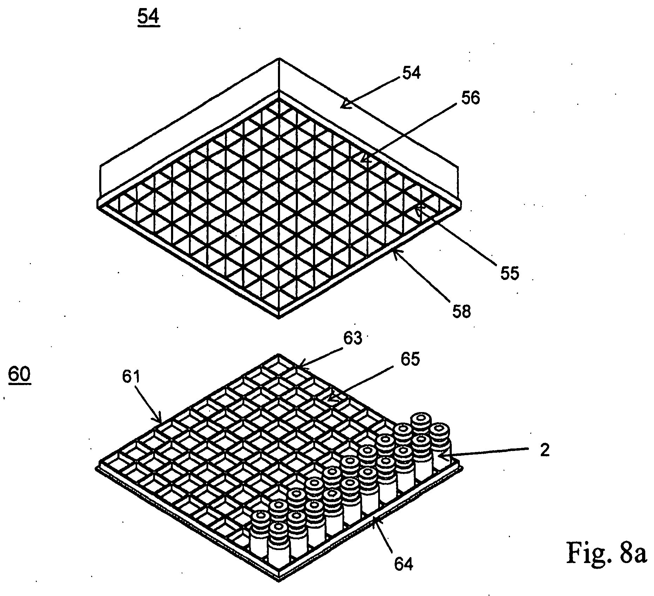

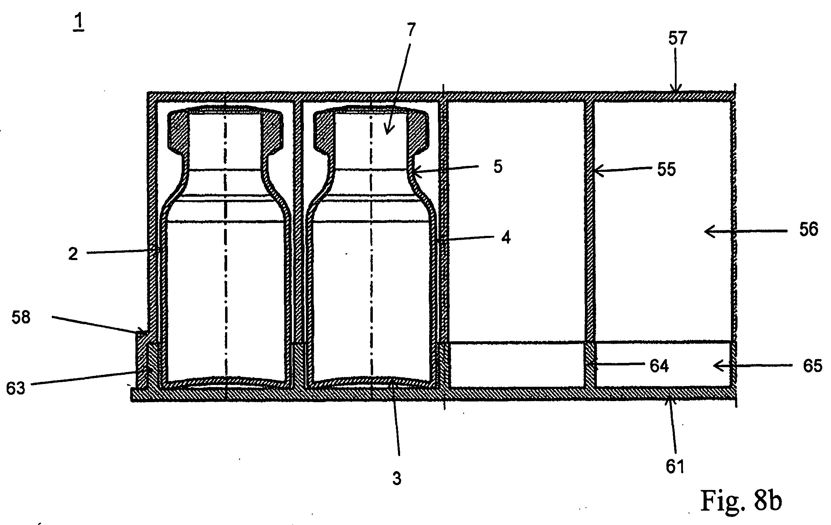

Die Fig. 8a zeigt eine weitere Variante, bei der der vorgenannte Randabschnitt der Grundplatte sehr schmal ausgebildet ist, so dass die Seitenwände 63 der Haltestruktur 60 unmittelbar in dem korrespondierend ausgebildeten Randabschnitt 58 des Einsatzes 54 aufgenommen sind, wie in der Schnittdarstellung gemäß der Fig. 8b dargestellt. Im zusammengesetzten Zustand gemäß der Fig. 8b ist ebenfalls eine Transport- und Verpackungseinheit 1 ausgebildet, die hermetisch gegen die Außenumgebung abgedichtet werden kann. Gemäß der Fig. 8b sind die Böden 3 der Behälter unmittelbar auf der Grundplatte 61 abgestützt und die Behälter 2 nahe ihrem unteren Ende in den von den Querstegen ausgebildeten länglichen Aufnahmen 65 reibschlüssig fixiert.

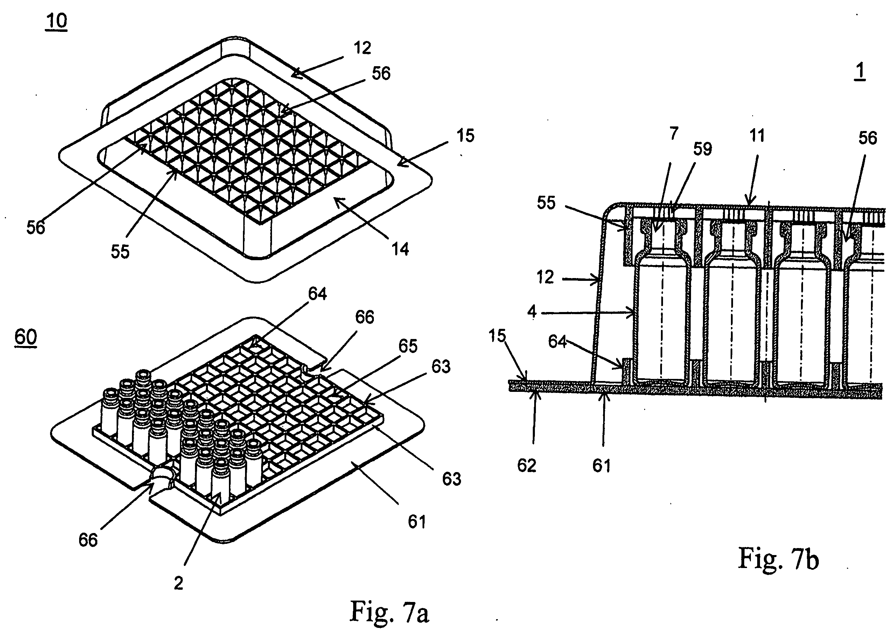

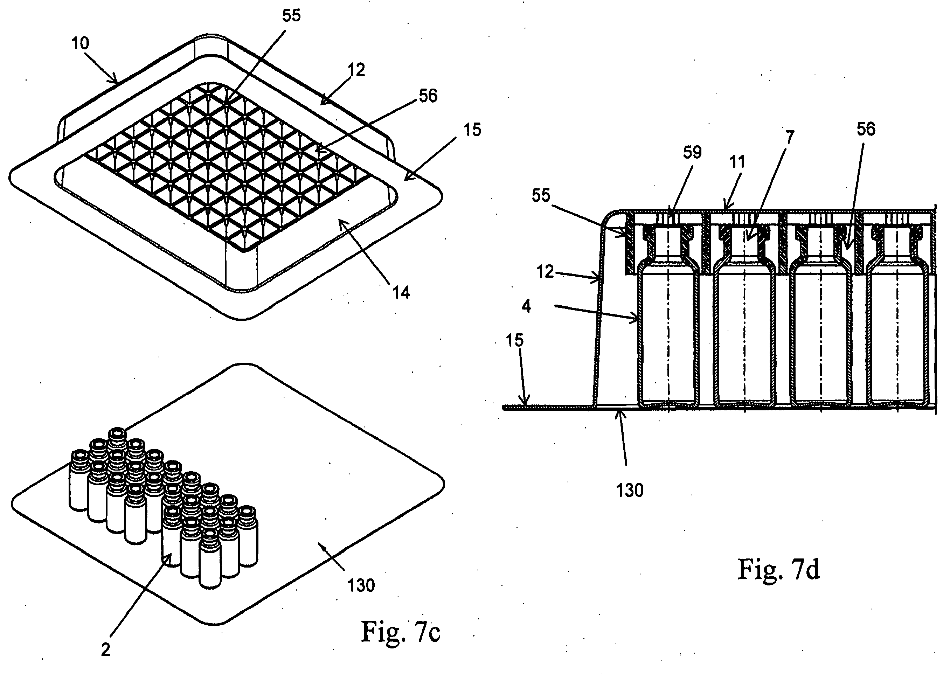

Bei einer weiteren Ausführungsform gemäß der Fig. 7a sind die Behälter 2 unmittelbar auf einer Schutz- oder Verpackungsfolie 130 angeordnet, auf die ein Transport- und Verpackungsbehälter 10, wie vorstehend anhand der Fig. 1a beispielhaft beschrieben, aufgesetzt wird, wobei die auf dem Boden des Behälters 10 angeordneten Querstege 55 eine unmittelbare Berührung der Seitenwände 4 der Behälter verhindern. An ihrem oberen Ende können die Behälter durch die Querstege 55 auch reibschlüssig fixiert werden, insbesondere geklemmt werden. Bei der Folie 130 kann es sich insbesondere um eine sterile, jedoch gasdurchlässige Folie handeln, insbesondere um ein Kunststoff-Geflecht, wie beispielsweise Tyveck^{®}.

Die Figuren 7c bis 7e zeigen eine weitere Variante zu dieser Ausführungsform, bei der die in dem Transport- und Verpackungsbehälter 10 aufgenommenen Behälter durch Einblasen eines Gases durch die Folie 130 hindurch sterilisiert werden. Damit einströmende Gase in den Innenraum der Behälter 2 einströmen können, sind zwischen dem Boden 11 des Transport- und Verpackungsbehälters 10 und dem oberen Rand der Behälter 2 Abstandshalter 59 vorgesehen, sodass die Behälter nicht unmittelbar auf dem Boden 11 aufliegen.

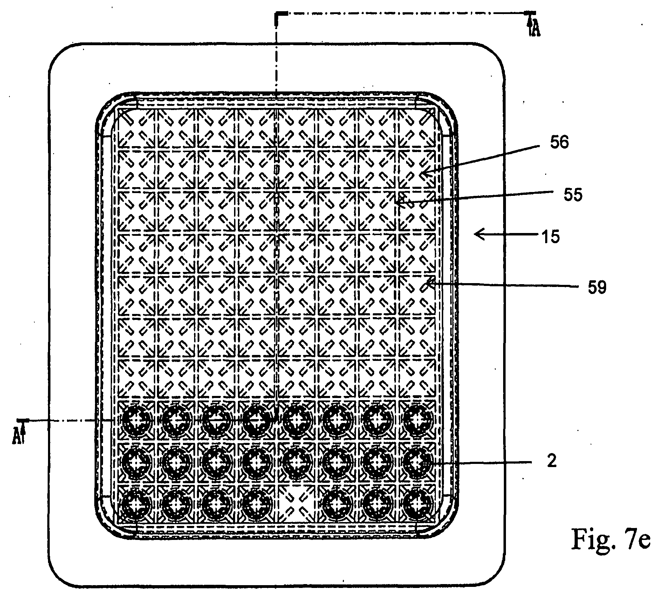

Diese Abstandshalter 59 können sich ausgehend von den Ecken einer jeweiligen Aufnahme 56 diagonal zur Mitte der jeweiligen Aufnahme 56 hin erstrecken. Die kreuzförmigen Abstandshaltestege 59 sind jedoch nicht miteinander verbunden, sodass im Bereich der Mitte einer jeweiligen Aufnahme 56 der obere Rand der Behälter ungehindert zugänglich ist. Die Fig. 7e zeigt eine Draufsicht auf den auf dem Boden des Transport- und Verpackungsbehälters 10 ausgebildeten Einsatz, der auch herausnehmbar sein kann.

Gemäß einer weiteren Ausführungsform gemäß den Figuren 10a bis 10e wird zum gleichzeitigen Halten einer Mehrzahl von Behältern 2 eine flächige Transportplatte 25 bereitgestellt, die aus einem Kunststoff ausgebildet ist, beispielsweise ausgestanzt oder spritzgegossen ist, und die aus einer Mehrzahl von kreisförmigen Ringösen 30 besteht, die miteinander verbunden sind. Die Ringösen 30 sind ausreichend flexibel oder aufweitbar, so dass die Behälter 2 von oben oder von unten her in die Öffnungen der Ringösen 30 eingeführt werden können. Dadurch können eine Mehrzahl von Behältern 2 im Bereich ihrer verengten Halsabschnitte 5 reibschlüssig fixiert werden. Dies ist in den schematischen Längsschnitten gemäß den Figuren 10c bis 10e genauer dargestellt.

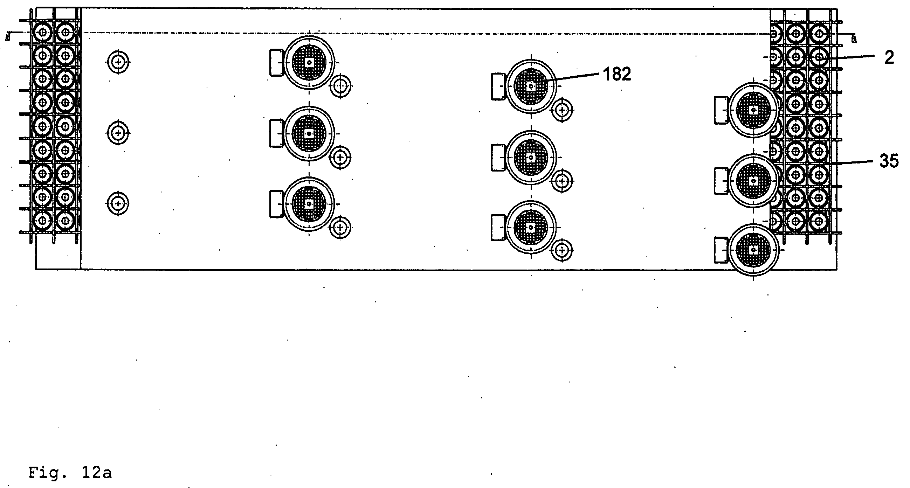

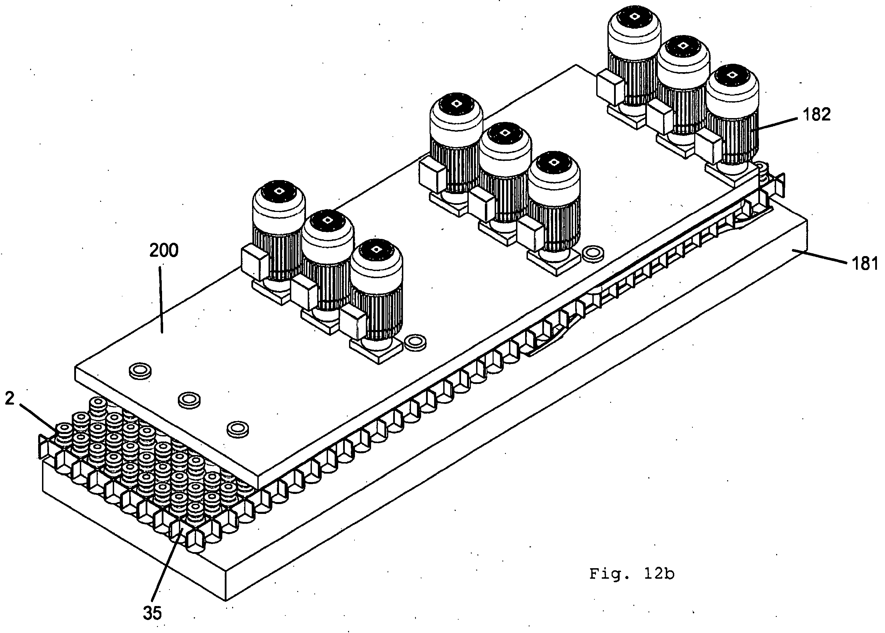

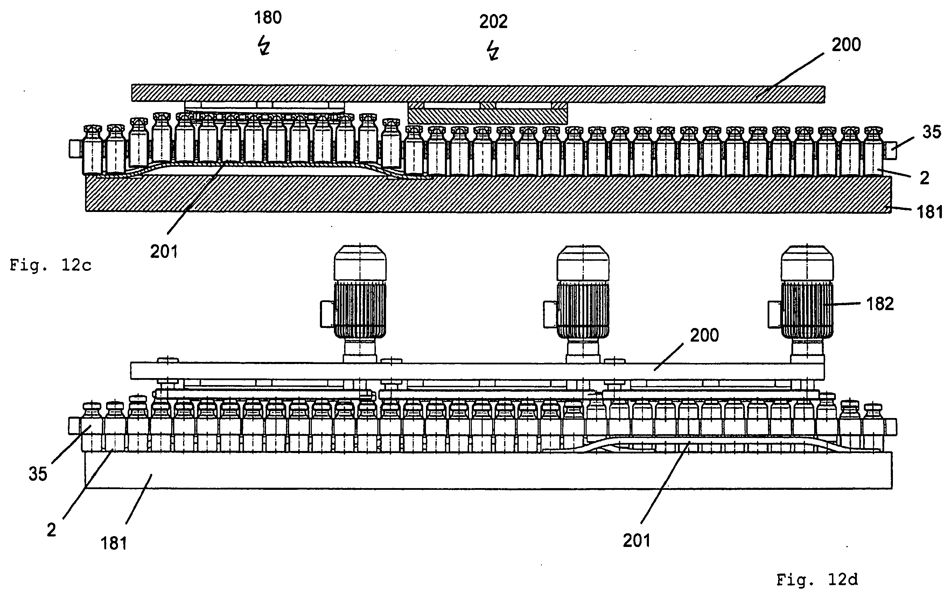

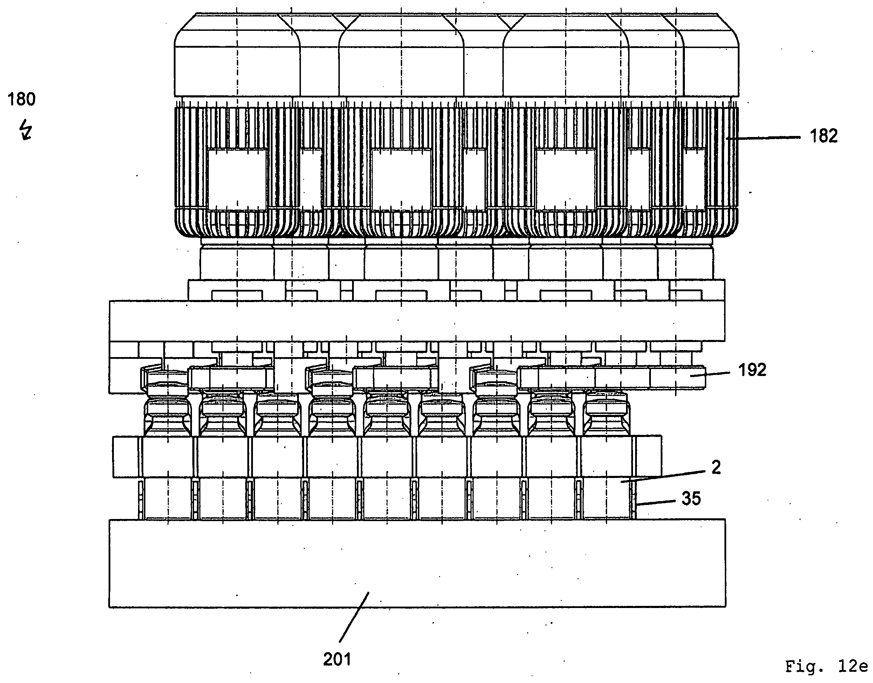

Bei den meisten vorvorstehend beschriebenen Ausführungsbeispielen sind die Böden der Behälter von der Unterseite her vollständig zugänglich, wenn diese gemeinsam an dem Träger gehalten werden. Dies ermöglicht beispielsweise die chargenweise Gefriertrocknung einer Mehrzahl von Behältern in einem Gefriertrockenschrank, während diese gemeinsam an dem Träger gehalten sind. Dies wird nachfolgend anhand der Figuren 12 bis 14 näher beschrieben werden.

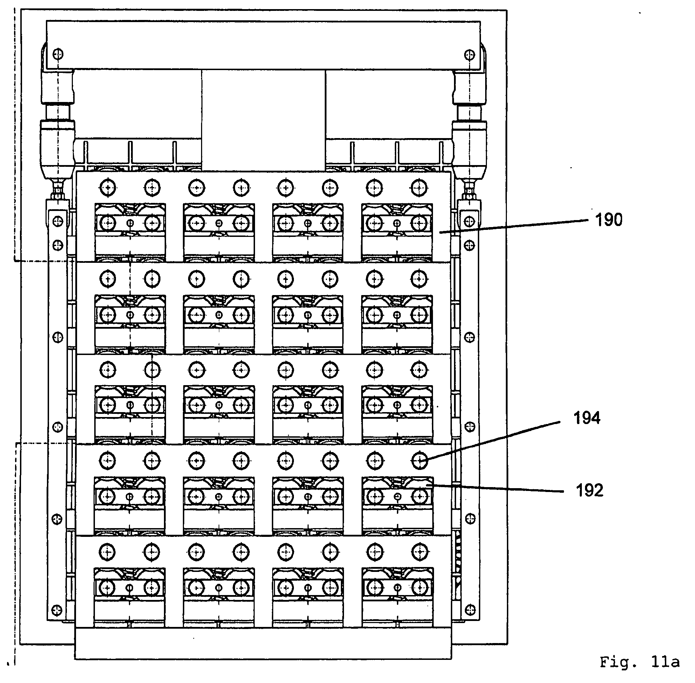

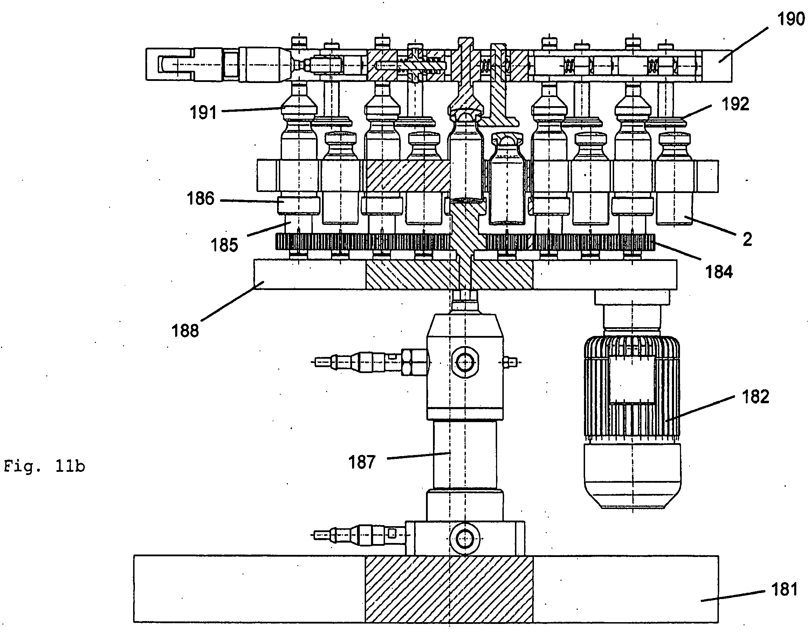

Die Figuren 11a und 11b zeigen eine weitere Variante für eine Bördelstation zum Bördeln eines Metalldeckels auf einem oberen Rand einer Mehrzahl von Behältern bei einem Verfahren gemäß der vorliegenden Erfindung. Die Zentrierscheiben 191 mit ihren Drehmitten 194 sind gemeinsam an einer rechteckförmigen Halteplatte 190 gelagert, an der auch die Bördelscheiben 192 gelagert sind, deren Positionen präzise justiert werden können. Bei dieser Variante werden sämtliche Behälter eines Trägers, oder eines Teilabschnittes hiervon, in zwei Prozessschritten gebördelt, da die Hubstangen 185 alternierend jeden zweiten der Behälter 2 anheben und absenken.

Die Figuren 12a bis 12d zeigen eine weitere Variante für eine Bördelstation 180 zum Bördeln eines Metalldeckels auf dem oberen Rand von Behältern bei einem Verfahren gemäß der vorliegenden Erfindung. Hier sind die Antriebsmotoren 182 mit zugeordneten Drehmitteln oberhalb der Ebene der Fördereinrichtung und der Träger angeordnet. Die Träger werden im Fließbandverfahren an der Bördelstation 180 vorbeigeführt, während diese von den Querstegen 35 der Träger reibschlüssig gehalten werden, zweckmäßig in der Stellung gemäß der Fig. 1a, in der der Reibschluss zwischen den Querstegen 35 und den Behältern 2 vollständig oder jedenfalls in ausreichendem Maße aufgehoben ist. Im Bereich der Bördelstation 180 erstreckt sich in Förderrichtung eine rampenartige Abstütz- und Führungsfläche 201, an welchen die Behälter 2 der Reihe nach in eine angehobene Position angehoben werden. Die lichte Weite zwischen dem unteren Ende der Querstege 35 und der Fördereinrichtung ist dabei ausreichend, sodass die Querstege 35 der Träger ungehindert die rampenartige Abstütz- und Führungsfläche 201 passieren können.

Wie man der vergrößerten Darstellung gemäß der Fig. 12e entnehmen kann, geraten die oberen Ränder der Behälter 2 in der angehobenen Stellung, also während deren Böden auf der rampenartigen Abstütz- und Führungsfläche 201 abgestützt sind, in den Einflussbereich der Bördelstation 180, sodass die auf das obere Ende der Behälter 2 aufgebrachten Metalldeckel geeignet gebördelt werden können.

Anschließend werden die Behälter 2 weiter entlang der rampenartigen Abstütz- und Führungsfläche 201 gefördert, bis diese schließlich wieder in die Normalstellung in die von den Querstegen 35 der Träger ausgebildeten Aufnahmen abgesenkt werden.

Ein entsprechendes Anheben und Absenken der Behälter, während diese in Trägern gehalten oder zumindest in diesen geführt sind, eignet sich vorteilhaft auch für die Inspektion der Behälter oder deren Inhalts, insbesondere mittels optischer Prüfverfahren. Wie man den meisten Darstellungen dieser Anmeldung entnehmen kann, ragen die Behälter mit ihrem oberen Rand zumeist nicht über den oberen Rand des zugeordneten Trägers hinaus, sodass diese nicht optisch geprüft und beurteilt werden können. Dies ist zumeist auch nicht zuverlässig möglich, wenn der Transport- und Verpackungsbehälter aus einem transparenten Kunststoff ausgebildet ist. In der vorstehend beschriebenen angehobenen Stellung sind die Behälter jedoch zumindest in deren oberem Bereich für ein Prüfverfahren oder zur Begutachtung zugänglich. Dies kann ausgenutzt werden, wenn man beispielsweise die Behälter in einer Fördereinrichtung mittels einer rampenartigen Führungsfläche, wie beispielhaft in den Figuren 12c und 12d dargestellt, die in die Bahn der Fördereinrichtung geeignet eingreift, gezielt anhebt und anschließend wieder zurück in die Aufnahmen der Träger absenkt.

Ein entsprechendes Anheben und Absenken der Behälter, während diese in Trägern gehalten oder zumindest in diesen geführt sind, wie vorstehend beschrieben, eignet sich auch vorteilhaft für eine koordinierte Übergabe der Behälter an nachgeordnete Prozessstationen. Beispielsweise können die Behälter einzeln oder reihenweise in der vorstehend beschriebenen angehobenen Stellung, in welcher die Haltekraft in den Aufnahmen oder Öffnungen des Trägers vollständig oder jedenfalls in ausreichendem Maße für deren Entnahme aufgehoben ist, mit einem Greifer gegriffen werden, beispielsweise an deren oberem Rand, und koordiniert übergeben werden.

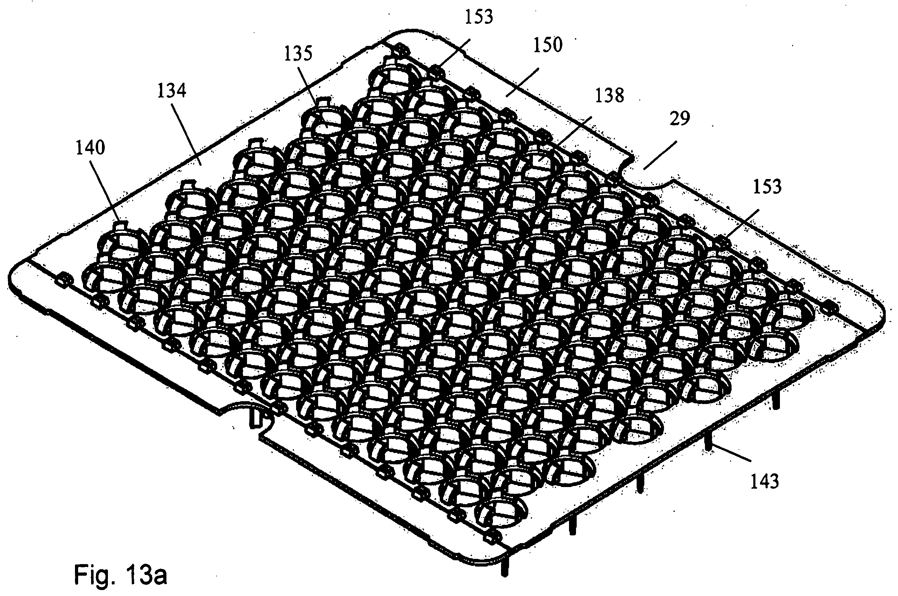

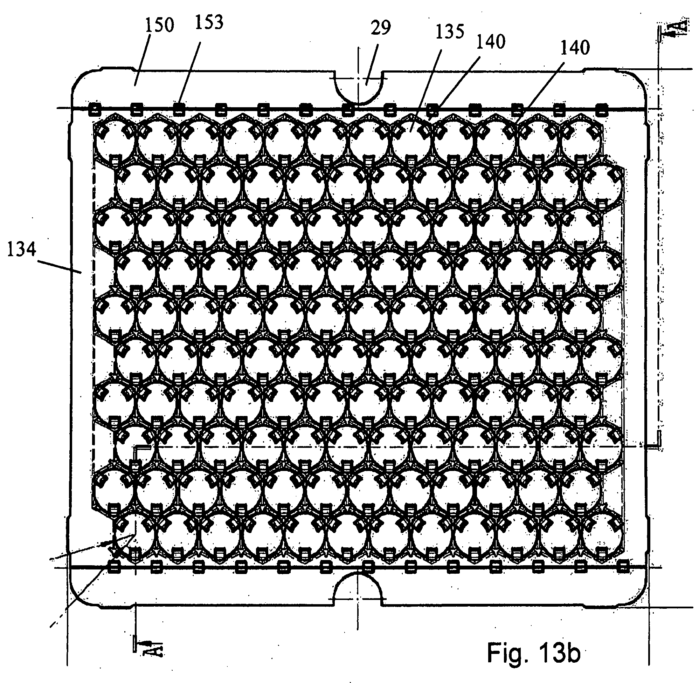

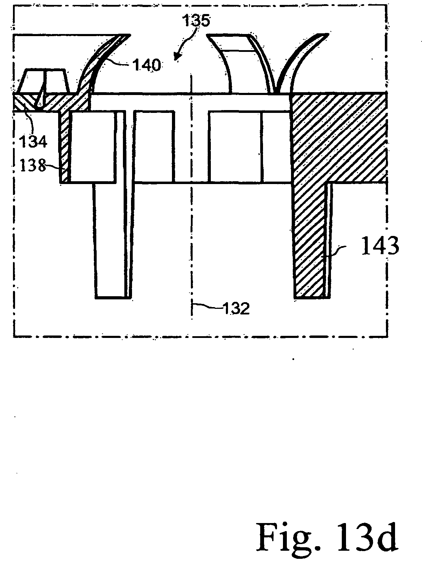

Zum gleichzeitigen Halten einer Mehrzahl von Behältern wird gemäß einer weiteren Ausführungsform der vorliegenden Erfindung, wie in den Figuren 13a und 13b dargestellt, ein flächiger rechteckförmiger Träger 134 bereitgestellt, der aus einem Kunststoff ausgebildet ist, beispielsweise ausgestanzt oder spritzgegossen ist, und eine Mehrzahl von Öffnungen 135 zur Aufnahme der Glasfläschchen 2 aufweist. Die Öffnungen 135 sind von Seitenwänden 138 (vgl. Fig. 13d) auf der Unterseite des Trägers 134 begrenzt. Gemäß der Fig. 13b ragen elastische Haltezungen 140 von der Oberseite des Trägers 134 bogenförmig ab und, in Draufsicht betrachtet, in die zugeordneten Öffnungen 135 hinein. Die elastischen Haltezungen 140 und die Seitenwände 138 sind bevorzugt einstückig mit dem flächigen Träger 134 ausgebildet, beispielsweise durch ein 1K- oder 2K-Kunststoff-Spritzgussverfahren.

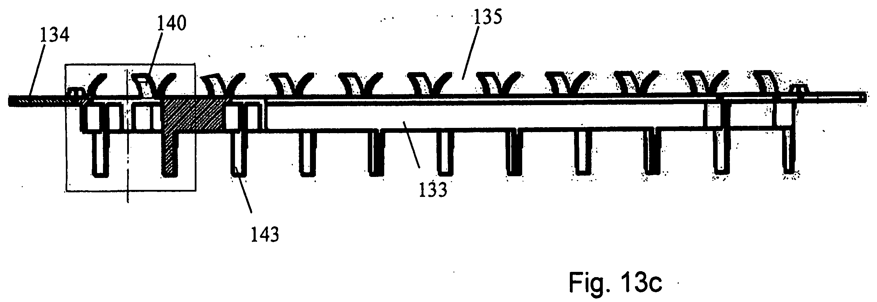

Wie man der Zusammenschau der Figuren 13b und 13d entnehmen kann, sind die Seitenwände 138 in einer regelmäßigen hexagonalen Anordnung auf der Unterseite des Trägers 134 verteilt angeordnet. Die Seitenwände 138 sind umlaufend ausgebildet, können jedoch als nur vergleichsweise kurze Seitenwandabschnitte ausgebildet sein, um eine zugeordnete Öffnung oder Aufnahme nur abschnittsweise zu begrenzen. In jedem Fall wird eine Kollision von Behältern, die in unmittelbar benachbarten Öffnungen 135 aufgenommen sind, durch die Seitenwände 138 verhindert. Gemäß der Fig. 13c ragen von der Unterseite des Trägers 134 Zapfen 143 ab, mit welchen der Träger 134 auf einer Ablagefläche und beabstandet zu dieser abgelegt werden kann.

Gemäß der Fig. 13b laufen die Seitenwände 138 jeweils in den Eckbereichen der Öffnungen 135 zusammen und sind dort miteinander verbunden oder einstückig ausgebildet. Von diesen Eckbereichen ragen die elastischen Haltezungen 140 in einer Anordnung mit dreizähliger Punktsymmetrie in die benachbarten Öffnungen 135 ab. Dies führt zu einer symmetrischen Kraftableitung beim Halten der Behälter über die Haltezungen 140. Die Haltezungen 140 bewirken so eine vorteilhafte Dreipunkt-Lagerung der Behälter in den Öffnungen, sodass die Behälter automatisch zentriert bezüglich einer Mittellinie 132 (vgl. Fig. 13d) einer jeweiligen Öffnung 135 gehalten werden.

Wie man der Fig. 13b entnehmen kann, ragen die Haltezungen 140 von den Seitenwänden 138 des Trägers 134 in Eckbereichen der Öffnungen 135 ab, also dort, wo die miteinander verbundenen oder einstückig ausgebildeten Seitenwände 138 Abschnitte mit vergleichsweise hoher Stabilität ausbilden. Zweckmäßig können in diesen Bereichen auch die vorgenannten Zapfen 143 angeformt bzw. ausgebildet sein.

Im Falle einer alternativen Ausführungsform, bei der die Seitenwände einer jeweiligen Öffnung oder Aufnahme jeweils kreisförmig und umlaufend ausgebildet sind, sind die Seitenwände ebenfalls bevorzugt miteinander verbunden oder einstückig ausgebildet. Die Haltezungen ragen dabei von den gleichen Bereichen ab wie bei der in der Fig. 13b dargestellten Anordnung. In diesen Bereichen können die Zwischenräume zwischen den kreisrund ausgebildeten Seitenwänden auch ausgefüllt sein.

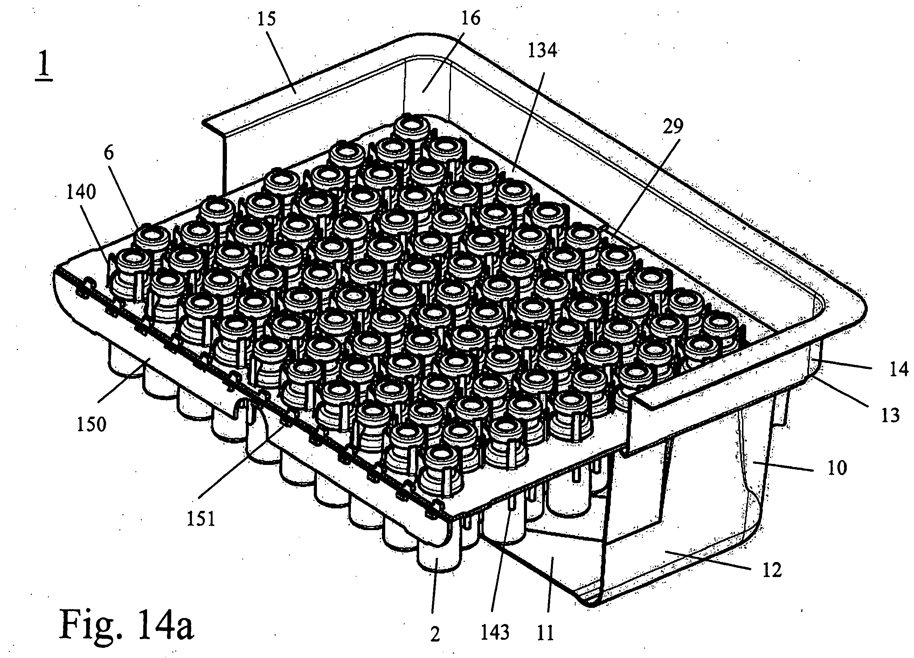

Die Fig. 13c zeigt einen Teilschnitt der Haltestruktur entlang A-A von Fig. 13b. Erkennbar ist, dass der Träger 134 auf der Unterseite von einem umlaufenden Rand 133 begrenzt ist, auf welchem der Träger 134 auf einer umlaufenden Stufe 13 (vgl. Fig. 14a) eines Transport- oder Verpackungsbehälter 1 abgestützt werden kann.

Die Fig. 13d zeigt einen stark vergrößerten Teilschnitt in dem Einsatz, der in der Fig. 13c dargestellt ist. Erkennbar ist, dass die Behälter ohne weiteres von unten her in die Öffnungen 135 des Trägers 134 eingeführt werden können. Beim Einführen der Behälter in die Öffnungen 135 kommt es zu einem elastischen Verbiegen der elastischen Haltezungen 140.

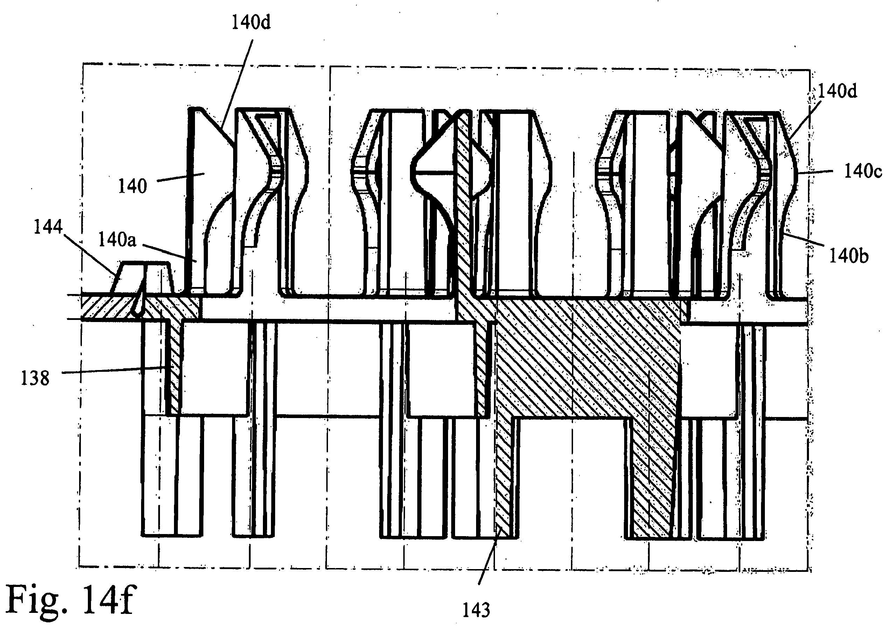

Je nach konkreter Ausgestaltung der zu haltenden Behälter können diese grundsätzlich auch von oben her in die Öffnungen 135 des Trägers 134 eingeführt werden, um an dem Träger 134 gehalten zu werden. Dies hat den Vorteil, dass das Risiko, dass Flüssigkeit oder anderer Behälterinhalt aus dem Behälterinnenraum der noch unverschlossenen Behälter unkontrolliert beim Einführen in die Öffnungen und beim Wegschwenken der Haltezungen 140 auf die Haltestruktur, insbesondere die Trägerplatte 134, gelangen können, noch weiter verringert werden kann. Zu diesem Zweck können auf der Oberseite der elastischen Haltezungen 140 Einführschrägen vorgesehen sein, wie diese nachfolgend näher anhand der Fig. 14f für eine alternative Ausführungsform beschrieben werden.

Durch Stärke, Material und Auslegung der elastischen Haltezungen 140 kann die zum Einführen und Entnehmen eines Behälters notwendige Kraft einfach vorgegeben werden.

Erfindungsgemäß sind die Behälter zumindest mit radialem Spiel und bevorzugt sowohl mit radialem als auch mit axialem Spiel lose auf den Haltezungen abgestützt. Auf diese Weise können auch große Toleranzen von Behältern und unterschiedliche Außendurchmesser im Bereich des Halsabschnitts 5 einfach ausgeglichen werden. Denn zur Halterung der Behälter genügt es, wenn der Rollrand 6 noch auf den Oberseiten der Haltezungen 140 aufliegt. Grundsätzlich können dadurch auch Behälter unterschiedlichen Typs, beispielsweise mit unterschiedlichen Durchmessern im Bereich des Halsabschnitts 5, von derselben Haltestruktur gehalten werden.

Die Fig. 13e verdeutlicht dies in dem gleichen stark vergrößerten Teilschnitt wie nach der Fig. 13d und stellt die Halterung eines Behälters in einer Öffnung 135 des Träges 134 dar. Gemäß der Fig. 13e liegt die Unterseite des verbreiterten Rands 6 auf dem vorderen Ende der elastischen Haltezungen 140 im Übergangsbereich zwischen dem verengten Halsabschnitt 5 und dem Rand 6 lose auf, um die Lage des Behälters zu fixieren. Wie man in der Fig. 13e erkennen kann, besteht zwischen den Haltezungen 140 (vgl. linker Bildteil) und dem verengten Halsabschnitt 5 ein Luftspalt, der ein radiales Spiel ermöglicht. Aufgrund dieser Halterung mit radialem Spiel besteht dabei, je nach konkreter Ausbildung des Behälters, noch die Möglichkeit, den von den Haltezungen 140 gehaltenen Behälter axial zu verschieben, d.h. in Längsrichtung der Behälter, beispielsweise solange, bis die Böden 3 von allen von dem Träger 134 gehaltenen Behältern unter dem gleichen Abstand zum Träger 134 gehalten werden, um gemeinsam eine Ebene aufzuspannen.

Gemäß der Fig. 13e ist der Behälter soweit in die Öffnung 135 eingeschoben, dass der verbreiterte Rand 6 auf den vorderen Enden der Haltezungen genau an dem Übergangsbereich zwischen dem verengten Halsabschnitt 5 und dem verbreiterten oberen Rand 6 abgestützt ist. Dies lässt sich beispielsweise durch Einschieben der Behälter von unten her in die Öffnungen 135 des Trägers 134 und anschließendes Herabdrücken der Behälter bewerkstelligen, und zwar solange, bis die vorderen Enden der Haltezungen genau an dem Übergangsbereich zwischen dem verengten Halsabschnitt 5 und dem verbreiterten oberen Rand 6 anliegen. In der Haltestellung gemäß der Fig. 13e ist jedenfalls bei der großen Mehrzahl der fixierten Behälter ein gewisser radialer Abstand zwischen dem stufenartigen Übergangsbereich zwischen dem oberen Rand 6 und dem verengten Halsabschnitt 5 und vorderen Ende der Haltezungen 140 vorgesehen. Auf diese Weise können in einem gewissen Umfang Fertigungstoleranzen der Behälter in axialer Richtung und auch Fertigungstoleranzen in radialer Richtung kompensiert werden und somit auch Behälter mit unterschiedlichen Durchmessern im Bereich des verengten halsabschnitt 5 von ein- und demselben Träger 134 gehalten werden. Damit lassen sich auch etwaige Verspannungen im Kunststoff des Trägers 134 aufgrund der Aufnahme von Behältern mit einem zu großen Außendurchmesser gering halten.

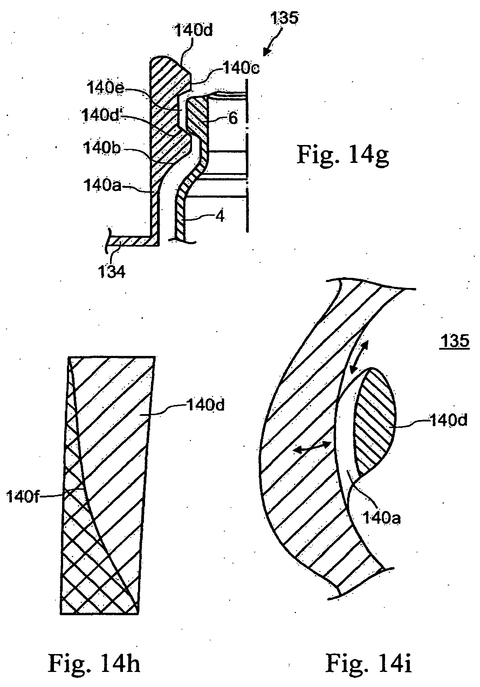

Gemäß einer alternativen Ausführungsform, wie nachfolgend anhand der Fig. 14g beschrieben, können die Behälter auch formschlüssig an dem Träger 134 gehalten werden.

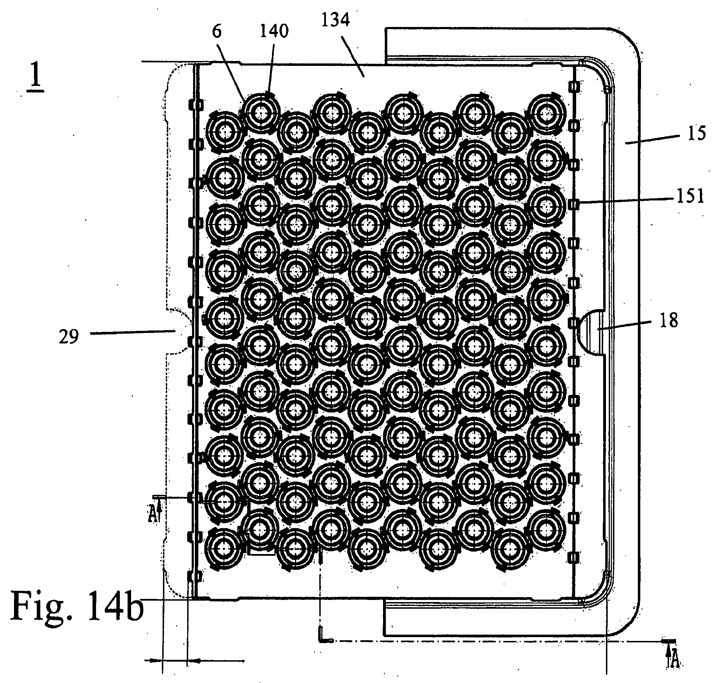

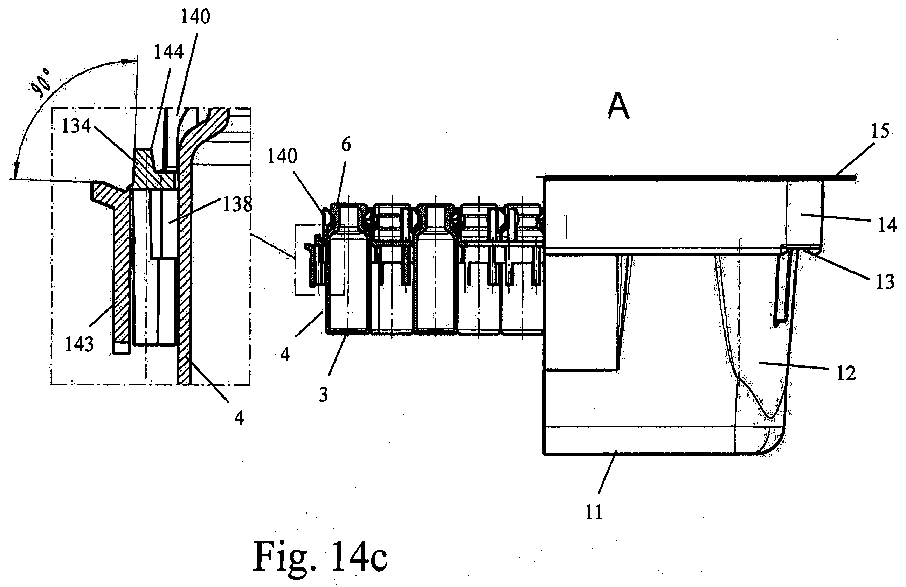

Zum Transport und zur Verpackung der vorstehend beschriebenen Haltestruktur mit den darin aufgenommenen Behältern dient ein Transport- und Verpackungsbehälter 10 (im Stand der Technik auch als "Tub" bezeichnet), wie dieser grundsätzlich anhand der Fig. 14c vorstehend beschrieben wurde und im Zusammenhang mit einer Darstellung einer weiteren Ausführungsform einer Haltestruktur gemäß der vorliegenden Erfindung in den Figuren 14a bis 14c gezeigt ist. Dabei zeigt die Fig. 14c in zwei vergrößerten Teilschnitten entlang A-A gemäß der Fig. 14b die Halterung von Behältern in der Haltestruktur gemäß der weiteren Ausführungsform sowie Details davon. Erkennbar ist insbesondere, dass auf der Oberseite des Trägers abgeschrägte Anschlagnasen 144 vorgesehen sind, welche das Zurückschwenken der elastischen Haltezungen 140 beim Einführen der Behälter begrenzen.

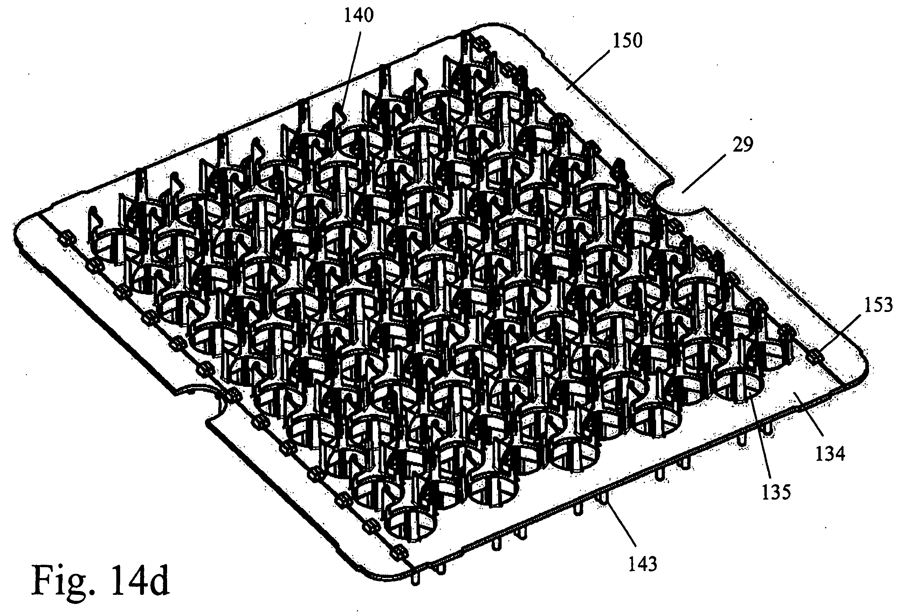

Die Fig. 14d zeigt in einer perspektivischen Draufsicht die Haltestruktur gemäß der Fig. 14a ohne Behälter. Erkennbar sind die elastischen Haltezungen 140 fähnchenartig und mit einer radial einwärts vorstehenden Haltenase ausgebildet, wie in dem stark vergrößerten Teilschnitt durch diese Haltestruktur gemäß der Fig. 14f besser dargestellt. Gemäß der Fig. 14f sind die elastischen Haltezungen 140 über eine senkrecht von der Oberseite des Trägers 134 abragende, elastische Basis 140a mit dem Träger 134 verbunden. Die Basis 140a geht in einen radial einwärts gekrümmten Abschnitt 140b über, der schließlich in die Haltenase 140c übergeht, auf welcher der verbreiterte Rand 6 (vgl. Fig. 13e) der Behälter aufliegt, wie vorstehend anhand der Fig. 13e für die erste Ausführungsform beschrieben. Die Haltenase 140c ragt dabei in die Öffnung des Trägers 134 hinein. Die Haltenase 140c geht in eine sich schräg aufwärts erstreckende Einführschräge 140d über, die mit dem oberen Ende der Haltezunge 140 verbindet. Aufgrund der Einführschräge 140d auf der Oberseite der Haltezunge 140 sowie des nach unten geöffneten, gekrümmten Abschnitts 140b der Haltezunge 140 können die Behälter wahlweise von oben oder von unten her in die Öffnungen des Trägers 134 eingeführt und aus diesen wieder abgezogen werden.

Beim Einführen der Behälter von oben her in die Öffnungen geraten zunächst die Böden bzw. unteren Enden der Behälter in Anlage zu den Einführschrägen 140d der Haltezungen 140. Beim weiteren Einführen der Behälter gleitet das untere Ende bzw. der Boden der Behälter entlang den Einführschrägen 140d abwärts und spreizt dabei die Haltezungen 140 zunehmend elastisch auseinander oder klappt bzw. schwenkt diese zurück. Beim weiteren Einführen der Behälter gerät schließlich die zylindrische Seitenwand der Behälter (vgl. Fig. 13e) in Anlage zu den Haltenasen 140c und gleitet an diesen entlang, solange bis schließlich die Unterseite des verbreiterten Rands der Behälter lose auf den Haltenasen 140c der Haltezungen 140 aufliegt. Die Behälter können dann entweder nach oben hin mit umgekehrtem Bewegungsablauf der Haltezungen 140 und ohne elastisches Verbiegen der Haltezungen 140 oder nach unten hin mit elastischem Verbiegen der Haltezungen 140 aus den Öffnungen des Trägers 134 entnommen werden.

Beim Einführen der Behälter von unten her in die Öffnungen gerät das obere Ende der Behälter zunächst in Anlage zu dem gekrümmten Abschnitt 140b der Haltezungen. Beim weiteren Einführen der Behälter gleitet das obere Ende der Behälter entlang den gekrümmten Abschnitten 140b aufwärts und spreizt dabei die Haltezungen 140 zunehmend elastisch auseinander oder klappt bzw. schwenkt diese zurück, bis schließlich die Haltenasen 140c erreicht sind. Beim weiteren Hochschieben der Behälter gleitet die Unterseite des verbreiterten Rands der Behälter über die Haltenasen 140c der Haltezungen 140 und liegt schließlich lose auf den Haltenasen 140c der Haltezungen 140 auf. Die Behälter können dann entweder nach unten hin mit umgekehrtem Bewegungsablauf der Haltezungen 140 und mit elastischem Verbiegen der Haltezungen 140 oder nach oben hin ohne elastisches Verbiegen der Haltezungen 140 aus den Öffnungen des Trägers 134 entnommen werden.

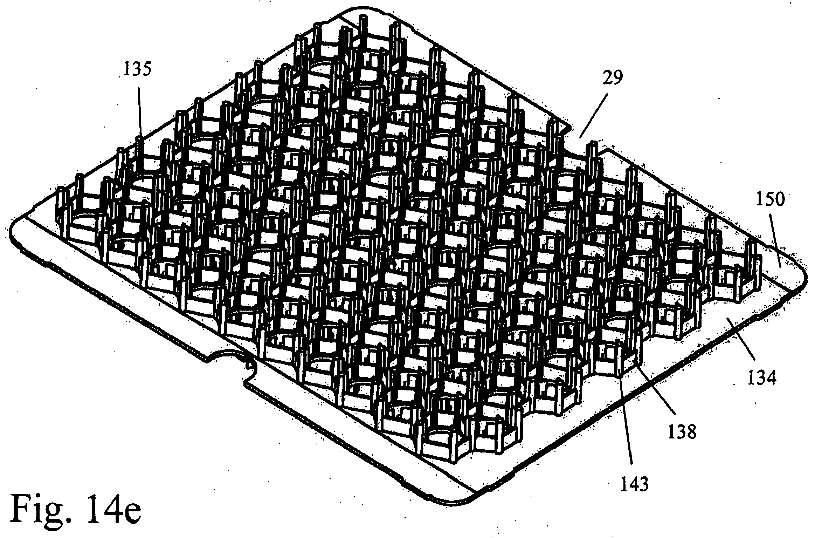

Die Fig. 14e zeigt in einer perspektivischen Unteransicht die Haltestruktur gemäß der Fig. 14a ohne Behälter. Erkennbar ist die wabenförmige, hexagonale Anordnung der umlaufenden Seitenwände 138, in deren Eckbereichen Zapfen 143 senkrecht von der Unterseite des Trägers 134 abragen. Diese Zapfen 143 dienen als Abstandshalter beim Ablegen des Trägers 134 auf einer Ablagefläche, beispielsweise dem Boden 11 eines Transport- und Verpackungsbehälters (vgl. Fig. 14a), vermeiden aber gleichzeitig auch den Kontakt der Behälter untereinander.

Die Fig. 14g zeigt in einem stark vergrößerten Teilschnitt die Halterung eines Behälters in einer Haltestruktur gemäß einer weiteren Ausführungsform der vorliegenden Erfindung. Abweichend zur vorgenannten Ausführungsform werden die Behälter hier an ihrem verbreiterten oberen Randabschnitt 6 (Rollrand) formschlüssig umgriffen, wobei ein ausreichendes radiales Spiel, wie vorstehend beschrieben, gewährleistet ist, wie durch den Luftspalt in Radialrichtung in der Fig. 14g angedeutet. Alternativ kann zusätzlich zu diesem radialen Spiel auch ein ausreichendes axiales Spiel gewährleistet sein, wie durch den Luftspalt in axialer Richtung in der Fig. 14g angedeutet. Zu diesem Zweck ist am vorderen Ende der Haltenase 140c (vgl. Fig. 14f) eine C-förmige Aussparung 140e vorgesehen, die über Schrägen 140d' in die Haltenase 140c übergeht. In der Haltestellung gemäß der Fig. 14g liegt der verbreiterte Randabschnitt 6 lose und mit radialem Spiel auf der unteren Schräge 140d' der Aussparung 140e auf. Wie in der Fig. 14g dargestellt, kann zwischen dem oberen Ende des verbreiterten Randabschnitts 6 und der oberen Schräge 140d' der Aussparung ein ausreichendes axiales Spiel bereitgestellt sein. Insgesamt wird somit der verbreiterte Randabschnitt 6 von der Haltezunge 140 klammerartig und formschlüssig umgriffen. Die Einführschräge 140d', der gekrümmte Abschnitt 140b sowie die Schrägen 140d' der Aussparung ermöglichen dabei ein Einführen und Abziehen der Behälter ohne größeren Kraftaufwand in die Öffnungen bzw. aus diesen heraus unter elastischem Wegbiegen der Haltezungen 140.

Die Fig. 14h stellt eine stark vergrößerte Draufsicht auf eine Einführschräge einer Haltezunge gemäß einer Variante zu der Haltestruktur gemäß der Fig. 14a dar. Gemäß der Fig. 14h ist die Einführschräge 140d mittels eines darauf ausgebildeten bogenförmigen Grats 140f insgesamt verdrillt ausgebildet. Diese gewundene Einführschräge 140d ist auf sämtlichen Haltezungen der Öffnungen oder Aufnahmen gleich ausgebildet. Insgesamt sind die Einführschrägen, in Draufsicht betrachtet, um einen Winkel von kleiner 90° gekrümmt ausgebildet. Im Zusammenwirken mit dem Behälter bewirkt dies beim Einführen der Behälter in die Öffnungen, dass die Haltezungen nicht nur radial auswärts weggeschwenkt oder zurück geklappt werden, sondern gleichzeitig mit einer Bewegungskomponente in Umfangsrichtung in Entsprechung zu der Geometrie der Einführschrägen 140d weggedreht werden, und zwar um einen Winkel von kleiner 90°. Je nach der Geometrie der Anordnung der Haltezungen auf dem Träger kann so eine Kollision von Haltezungen von unmittelbar benachbarten Öffnungen oder Aufnahmen beim Zurückschwenken oder Zurückklappen vermieden werden. Auf diese Weise kann die Packungsdichte der Behälter an der Haltestruktur noch weiter erhöht werden.

Die Fig. 14i zeigt in einer Draufsicht eine weitere Variante von Haltezungen für eine Haltestruktur gemäß der Fig. 14a, bei der die Basis 140a, in Axialrichtung betrachtet, verdrillt ausgebildet ist, was im Zusammenwirken der Einführschräge 140d mit dem Behälter beim Einführen des Behälters von oben her in die Öffnung oder Aufnahme sowohl eine radiale Komponente als auch eine Komponente in Umfangsrichtung beim elastischen Wegschwenken der Haltezungen ergibt, wie durch die beiden Doppelpfeile schematisch angedeutet.

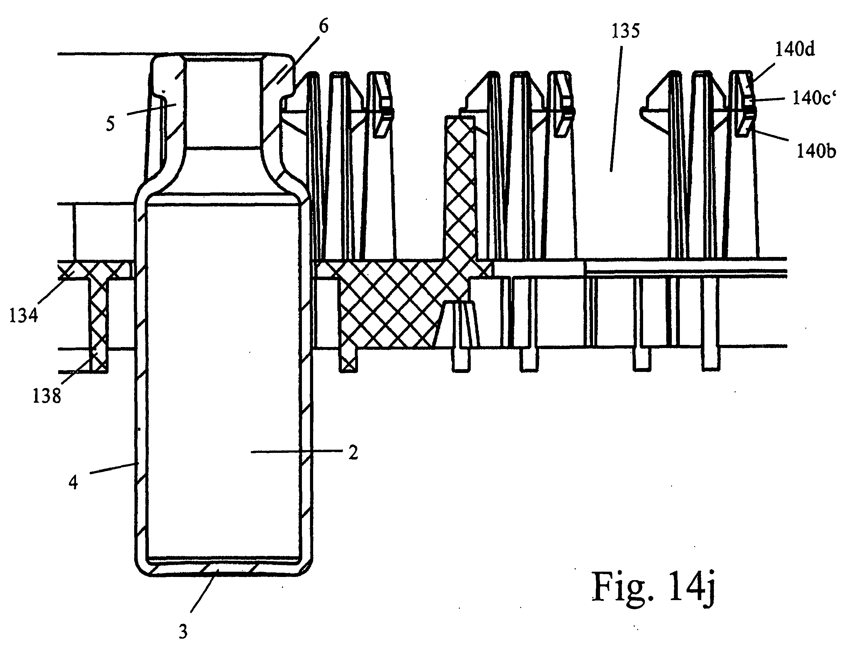

Die Fig. 14j zeigt in einer schematischen Schnittansicht eine weitere Variante von Haltezungen für eine Haltestruktur gemäß der Fig. 14a, bei der unterhalb der oberen Einführschräge 140d eine sich im Wesentlichen senkrecht erstreckende Aussparung 140c' ausgebildet ist, welche in eine Stufe übergeht, auf der die Unterseite des oberen Rands des aufzunehmenden Behälters unmittelbar aufliegt. Der Stufe schließt sich die untere Einführschräge 140b in der vorstehend beschriebenen Weise an.

Wie dem Fachmann beim Studium der vorstehenden Beschreibung ohne weiteres ersichtlich sein wird, ist der vorgenannte Gesichtspunkt der form- oder kraftschlüssigen Verbindung von unmittelbar benachbarten Haltestrukturen grundsätzlich unabhängig von der konkreten Ausführung der Halterung der Fläschchen an solchen Haltestrukturen, sodass dieser Gesichtspunkt grundsätzlich auch als unabhängiger Gesichtspunkt der vorliegenden Erfindung unabhängig von der konkreten Realisierung der Halterung der Fläschchen an solchen Haltestrukturen beansprucht werden kann.

Das Verfahren nach der Erfindung beruht maßgeblich darauf, dass eine Mehrzahl von Behältern gemeinsam an einem Träger gehalten werden und, während diese an dem Träger gehalten oder zumindest geführt sind, behandelt oder weiter verarbeitet werden können. Wie dem Fachmann bei Studium der vorstehenden Beschreibung ohne Weiteres ersichtlich sein wird, eignet sich dieser Lösungsansatz grundsätzlich für beliebige Prozessschritte zur Behandlung oder Verarbeitung von Behältern zur Aufbewahrung von Substanzen für kosmetische, medizinische oder pharmazeutische Anwendungen.

Die von den reibschlüssig oder formschlüssig wirkenden Haltemitteln eines Trägers jeweils auf die Behälter ausgeübte Haltekraft ist ausreichend, um die Behälter zuverlässig an der Haltestruktur zu halten. Insbesondere ist die ausgeübte Haltekraft größer als die Gewichtskraft der Behälter, ggf. mit Inhalt und Verschlussstopfen. Gemäß weiteren Ausführungsformen kann die Haltekraft durch geeignete Auslegung der Haltemittel auch so bemessen sein, dass diese größer ist als übliche Kräfte bei der Handhabung, Verarbeitung oder Behandlung der Behälter in einer Prozessanlage. Dadurch wird stets eine zuverlässige Halterung der Behälter gewährleistet. Gleichwohl können die Behälter gemäß bevorzugten weiteren Ausführungsformen der Erfindung gegen diese Haltekraft in den Öffnungen oder Aufnahmen verstellt werden, insbesondere axial vorgeschoben oder gedreht werden. Die hierfür erforderliche Kraft muss nur größer sein als die von den Haltemitteln ausgeübte Kraft.

Zum Einführen, Herausnehmen oder Verschieben der Behälter an einem Träger muss diese Haltekraft zunächst überwunden werden. Dies hat den Vorteil, dass die Behälter bei Einwirkung einer geringen Kraft, etwa durch Erschütterungen der Prozessanlage oder von Fördermitteln derselben, weiterhin zuverlässig an dem Träger gehalten werden und nicht etwa versehentlich umfallen. Dies mindert die Gefahr von unbeabsichtigten Verunreinigungen in Prozessanlagen erheblich. So kleben in die Öffnung von Behältern eingebrachte Stopfen häufig im Laufe einer Behandlung an und können danach nicht rüttelfrei verstellt werden, beispielsweise um einen Behälter zu verschließen oder diesen zu öffnen. Herkömmlich hat dies häufig zu einem Umfallen von Behältern und zu einem unerwünschten Auslaufen von Substanzen in Prozessanlagen geführt. Da erfindungsgemäß die Behälter mit einer vorbestimmten Minimum-Haltekraft an den Trägern gehalten sind, ist dieses Risiko bei einem Verfahren nach der Erfindung erheblich minimiert.

Herkömmlich wurden deshalb Stopfen oder vergleichbare elastische Verschlussteile mit einer Gleitbeschichtung versehen, was zu unerwünschten Verunreinigungen führte. Erfindungsgemäß kann grundsätzlich ohne solche Gleitbeschichtungen gearbeitet werden, sodass Wirkstoffe mit einem Verfahren nach der Erfindung in noch reinerer Form verarbeitet und behandelt werden können.

Selbstverständlich kann die Haltestruktur (der Träger) im Sinne der vorliegenden Erfindung auch aus einem thermoplastischen, duroplastischen oder elastomeren Kunststoff ausgebildet sein, zumindest Abschnitte der Haltestruktur bzw. des Trägers mit einer reibreduzierenden Beschichtung versehen werden, um das Einführen und die Entnahme der Behälter zu erleichtern.

Gemäß weiteren Ausführungsformen kann die Haltestruktur und/oder der Transportbehälter, oder Abschnitte davon, aus einem faserverstärkten Kunststoffe oder einem Kunststoff ausgebildet sein, dem zur Erhöhung seiner Wärmeleitfähigkeit Keramiken oder Metalle beigegeben beigegeben sind. Bekanntermaßen haben faserverstärkte Kunststoffe eine höhere Wärmeleitfähigkeit bis 0,9 W/(K m) bei Kohlenstofffasern. Werden den Kunststoffen Keramiken oder Metalle beigegeben, so wird die Wärmeleitfähigkeit weiter vergrößert. Es entstehen die sogenannten wärmeleitenden Kunststoffe. So wird eine Wärmeleitfähigkeit von 20 W/(K m) erreicht.

Wie dem Fachmann beim Studium der vorstehenden Beschreibung ohne Weiteres ersichtlich sein wird, können die einzelnen Gesichtspunkte und Merkmale der vorstehend beschriebenen Ausführungsbeispiele auch in beliebiger Weise miteinander kombiniert werden, was in zahlreichen weiteren Ausführungsformen und Modifikationen resultiert. Wie dem Fachmann beim Studium der vorstehenden Beschreibung ohne weiteres ersichtlich sein wird, sollen sämtliche solche weiteren Ausführungsformen und Modifikationen von der vorliegenden Erfindung mit umfasst sein, solange diese nicht von dem allgemeinen Lösungsgedanken und dem Schutzbereich der vorliegenden Erfindung abweichen, wie in den beigefügten Patentansprüchen festgelegt.

## Patentansprüche

1. Verfahren zur Behandlung oder Verarbeitung von Behältern (2), die zur Aufbewahrung von Substanzen für medizinische, pharmazeutische oder kosmetische Anwendungen dienen oder diese enthalten, wobei die Behälter mittels einer Fördereinrichtung zur Behandlung oder Verarbeitung automatisiert an Prozessstationen vorbeigeführt werden oder diese durchlaufen, bei welchem Verfahren
eine Mehrzahl von Behältern (2) von der Fördereinrichtung gefördert wird, während diese von einem Träger (25; 134) gemeinsam in einer regelmäßigen zweidimensionalen Anordnung gehalten werden, wobei der Träger eine Mehrzahl von Öffnungen oder Aufnahmen (32; 39; 87; 120) aufweist, welche die regelmäßige Anordnung vorgeben, und
die Behandlung oder Verarbeitung der Behälter an oder in zumindest einer der Prozessstationen ausgeführt wird, während diese von dem Träger gehalten werden oder in Öffnungen oder Aufnahmen des Trägers aufgenommen sind,
**dadurch gekennzeichnet, dass** bei dem Verfahren mittels einer Bördeleinrichtung (180) auf dem oberen Rand der Behälter ein Metalldeckel gebördelt wird, wobei
die Behälter zum Bördeln in der jeweiligen Öffnung oder Aufnahme in Längsrichtung in eine angehobene Position verschoben werden und in der angehobenen Position von einem Drehteller um ihre Längsachse gedreht werden, während die Behälter in den Öffnungen oder Aufnahmen des Trägers aufgenommen sind, und
nach dem Bördeln wieder in die Öffnungen oder Aufnahmen zurückgeschoben werden, um von dem Träger gehalten zu werden.

2. Verfahren nach Anspruch 1, wobei die Behälter (2) zum Bördeln jeweils mittels einer Hubstange (185) in die angehobenen Position angehoben werden, wobei der jeweilige Drehteller (194) auf der Hubstange gelagert ist und wobei der obere Rand (6) der Behälter (2) mit dem darauf aufgesetzten Metalldeckel (193) während des Drehens mittels einer Zentrierscheibe (191) zentriert wird.

3. Verfahren nach Anspruch 1 oder 2, wobei die Behälter (2) zur Behandlung oder Verarbeitung an oder in der Prozessstation in der jeweiligen Öffnung oder Aufnahme in Längsrichtung in eine angehobene Position verschoben werden.

4. Verfahren nach Anspruch 3, wobei die Behälter in der angehobenen Position weiterhin in den Öffnungen oder Aufnahmen des Trägers gehalten werden, um an oder in der Prozessstation behandelt oder verarbeitet zu werden.

5. Verfahren nach Anspruch 3, wobei die Behälter in der angehobenen Position weiterhin in den Öffnungen oder Aufnahmen des Trägers aufgenommen sind, jedoch auf einer zusätzlichen Abstützfläche abgestützt werden oder mittels einer zusätzlichen Halte- oder Greifeinrichtung gehalten werden, um an oder in der Prozessstation behandelt oder verarbeitet zu werden.

6. Verfahren nach Anspruch 5, wobei die zusätzliche Abstützfläche zumindest einen drehbeweglich gelagerten und angetriebenen Drehteller aufweist, auf welchem die Behälter während der Behandlung oder Verarbeitung an oder in der Prozessstation gedreht werden, während diese weiterhin in den Öffnungen oder Aufnahmen des Trägers aufgenommen sind.

7. Verfahren nach einem der vorhergehenden Ansprüche, wobei die Träger (25; 134) während der Behandlung oder Verarbeitung in oder an zumindest einer der Prozessstationen in Transport- und Verpackungsbehältern (1) angeordnet sind.

8. Verfahren nach einem der vorhergehenden Ansprüche, wobei den Öffnungen oder Aufnahmen des Trägers Haltemittel zugeordnet sind, welche die Mehrzahl von Behältern (2) an dem Träger an den vorbestimmten Positionen und in der regelmäßigen zweidimensionalen Anordnung halten, wobei die Haltemittel die Behälter (2) an dem Träger (25; 134) formschlüssig halten.

9. Verfahren nach Anspruch 8, wobei der Träger (25) eine Mehrzahl von sich parallel erstreckenden und unter regelmäßigen Abständen zueinander angeordneten Querstegen (165) umfasst, entlang denen unter regelmäßigen Abständen zueinander eine Mehrzahl Paaren von elastischen, konkav gewölbten Haltearmen (166) angeordnet sind, welche den oberen Rand (6) der von einem Paar von Haltearmen aufgenommenen Behälter (2) jeweils formschlüssig fixieren, wobei die Behälter unter Aufspreizung der Haltearme in diese eingeführt oder aus diesen abgezogen werden.

10. Verfahren nach Anspruch 8, wobei die Haltemittel zumindest zwei Haltezungen (140) umfassen, die am Rand einer jeweiligen Öffnung oder Aufnahme vorgesehen sind und von einer Oberseite des Trägers (134) abragen, um den jeweiligen Behälter zu halten, wobei die Haltezungen (140) beim Einführen der Behälter in die Öffnungen oder Aufnahmen elastisch weggeschwenkt oder weggeklappt werden, und wobei die Haltezungen (140) die Behälter mit radialem Spiel halten, wobei die Haltezungen (140) bevorzugt elastische Haltezungen sind.

11. Verfahren nach Anspruch 10, wobei die Haltezungen (140) die Behälter so halten, dass die Behälter (2) mit einem verbreiterten Rand (6), der an einem oberen Ende der Behälter ausgebildet ist, lose auf Oberseiten der Haltezungen (140) aufliegen, oder wobei die Haltezungen (140) einen verbreiterten Rand (6), der an einem oberen Ende der Behälter ausgebildet ist, so umgreifen, dass die Behälter mit radialem Spiel oder mit radialem und axialem Spiel von den Haltezungen gehalten werden, wobei am oberen Ende der Haltezungen (140) bevorzugt Einführschrägen (140d) ausgebildet sind, welche jeweils in eine von den Haltezungen radial einwärts vorstehende Haltenase (140e) zum Halten der Behälter übergehen

12. Verfahren nach einem der Ansprüche 1 bis 6, wobei den Öffnungen oder Aufnahmen des Trägers Haltemittel zugeordnet sind, welche die Mehrzahl von Behältern (2) an dem Träger an den vorbestimmten Positionen und in der regelmäßigen zweidimensionalen Anordnung halten, wobei die Haltemittel die Behälter (2) an dem Träger (25; 134) reibschlüssig halten.

13. Verfahren nach Anspruch 12, wobei die Haltemittel als umlaufende und sich in Längsrichtung der Behälter erstreckende Aufnahmen (39; 65) ausgebildet sind und die Seitenwandabschnitte (4) der Behälter (2) zumindest abschnittsweise reibschlüssig umgreifen, wobei die Aufnahmen einen vieleckigen Querschnitt aufweisen, insbesondere einen viereckigen oder sechseckigen Querschnitt, wobei der Reibschluss durch das Zusammenwirken von einander gegenüberliegenden Seitenwänden und der zylindrischen Seitenwand (4) der Behälter bewerkstelligt wird.

14. Verfahren nach Anspruch 13, wobei sämtliche Seitenwände der Öffnungen oder Aufnahmen zwischen einer ersten Stellung und einer zweiten Stellung koordiniert verstellbar sind, wobei die Behälter in der ersten Stellung mit geringem Kraftaufwand in die Öffnungen oder Aufnahmen einführbar oder in diesen verschiebbar sind und die Behälter in der zweiten Stellung reibschlüssig in den Öffnungen oder Aufnahmen fixiert werden.

15. Vorrichtung zur Behandlung oder Verarbeitung von Behältern (2), die zur Aufbewahrung von Substanzen für medizinische, pharmazeutische oder kosmetische Anwendungen dienen oder diese enthalten,
mit einer Fördereinrichtung; und
einer Mehrzahl von Prozessstationen, in oder an denen die Behälter (2) jeweils behandelt oder verarbeitet werden;
welche Vorrichtung zur Behandlung oder Verarbeitung von Behältern ausgelegt ist, sodass
eine Mehrzahl von Behältern (2) von der Fördereinrichtung zur Behandlung oder Verarbeitung automatisiert an den Prozessstationen vorbeigeführt werden oder diese durchlaufen, während diese von einem Träger (25; 134) gemeinsam in einer regelmäßigen zweidimensionalen Anordnung gehalten werden, wobei der Träger eine Mehrzahl von Öffnungen oder Aufnahmen (32; 39; 87; 120) aufweist, welche die regelmäßige Anordnung vorgeben, und
mit einer Bördeleinrichtung (180), bei der auf dem oberen Rand der Behälter ein Metalldeckel gebördelt wird,
**dadurch gekennzeichnet, dass** die Vorrichtung so ausgelegt ist, dass
die Behälter zum Bördeln in der jeweiligen Öffnung oder Aufnahme in Längsrichtung in eine angehobene Position verschoben werden und in der angehobenen Position von einem Drehteller um ihre Längsachse gedreht werden, während die Behälter in den Öffnungen oder Aufnahmen des Trägers aufgenommen sind, und
nach dem Bördeln wieder in die Öffnungen oder Aufnahmen zurückgeschoben werden.

16. Vorrichtung nach Anspruch 15, welche weiterhin so ausgelegt ist, dass die Behandlung oder Verarbeitung der Behälter an oder in der jeweiligen Prozessstation ausgeführt wird, während diese von dem Träger gehalten werden, wobei zumindest einige der Prozessstationen bevorzugt unter sterilen Umgebungsbedingungen angeordnet und betrieben werden.

## Claims

1. A process for the treatment or processing of containers (2), which serve for storing substances for medical, pharmaceutical or cosmetic applications or contain such substances, wherein the containers are conveyed automatically, by means of a conveyor, to processing stations or pass them for the treatment or processing, in which process
a plurality of containers (2) are conveyed by the conveyor while being supported by a carrier (25; 134) in a regular two-dimensional array, wherein the carrier comprises a plurality of openings or receptacles (32; 39; 87; 120), which define the regular array, and
the treatment or processing of the containers at or in at least one of the processing stations is carried out while being supported by said carrier or while being accommodated in openings or receptacles of said carrier,
**characterized in that** in the process a metal lid is crimped on the upper rim of the containers by means of a crimping device (180), wherein
the containers are displaced in the respective opening or receptacle in an axial direction to a raised position to be crimped and rotated by a turntable about a longitudinal axis thereof in said raised position while the containers are accommodated in the openings or receptacles of the carrier, and
are pushed back into the openings or receptacles to be supported by said carrier.

2. The process according to claim 1, wherein for said crimping the containers are lifted into the raised position by means of a lifting rod (185), wherein the respective turntable (194) is supported on the lifting rod and wherein the upper rim (6) of the containers (2) together with the metal lid (193) placed thereon is centered during rotation by means of a centering disc (191).

3. The process according to any of the preceding claims, wherein the containers (2) are displaced in the respective opening or receptacle in a longitudinal direction thereof to a raised position for the treatment or processing at or in the processing station.

4. The process according to claim 3, wherein the containers continue to be held in the raised position in the openings or receptacles of the carrier to be treated at or in the processing station.

5. The process according to claim 3, wherein the containers continue to be accommodated in the openings or receptacles in the raised position of the carrier, however, are supported on an additional supporting surface or by an additional holding or gripping device to be treated or processed at or in the processing station

6. The process according to claim 5, wherein the additional supporting surface comprises at least one rotatable and driven turntable on which the containers are rotated while being treated or processed at or in the processing station and while being accommodated in the openings or receptacles of the carrier.

7. The process according to any of the preceding claims, wherein the carriers (25; 134) are held in transport and packaging containers (1) during treatment or processing in or at at least one of the processing stations.

8. The process according to any of the preceding claims, wherein holding means are associated with the openings or receptacles of the carrier, which hold the plurality of containers (2) on the carrier at the predetermined positions and in the two-dimensional array, wherein said holding means support the containers (2) on the carrier (25; 134) in a positive-fit manner.

9. The process of claim 8, wherein the carrier (25) comprises a plurality of mutually-associated transverse webs (165) extending in parallel with each other and spaced apart from each other at regular intervals, along which a plurality of pairs of resilient, concavely shaped holding arms (166) are disposed which positively fix the upper rims (6) of the containers (2) accommodated by a pair of opposite holding arms (166), wherein the containers are inserted into or retracted from the holding arms while spreading the holding arms.

10. The process of claim 8, wherein the holding means comprises at least two holding tongues (140), which are disposed at a rim of the respective opening or receptacle and protrude from an upper side of the carrier (134) for supporting the respective container, wherein the holding tongues (140) are resiliently pivoted or clapped away when the containers are inserted into the openings or receptacles and wherein the holding tongues (140) support the containers with a radial play, wherein the holding tongues (140) are preferably resilient holding tongues.

11. The process of claim 10, wherein the holding tongues (140) support the containers such that the containers (2) rest loosely on upper sides of the holding tongues (140) with an upper rim (6) formed at an upper end of the containers, or
wherein the holding tongues (140) embrace an expanded rim (6) formed at an upper end of the containers such that the containers are supported by the holding tongues with a radial play or with a radial and axial play, wherein preferably slanted insertion surfaces (140d) are formed at upper ends of the holding tongues (140) each of which pass into a holding nose (140e) protruding inwards from the holding tongues for supporting the containers.

12. The process according to any of claims 1 to 6, wherein holding means are associated with the openings or receptacles of the carrier, which hold the plurality of containers (2) on the carrier at the predetermined positions and in the two-dimensional array, wherein said holding means are configured for holding the containers (2) on the carrier (25; 134) by friction.

13. The process of claim 12, wherein the holding means are formed as circumferential receptacles (39; 65) extending in the longitudinal direction of the containers and wherein side wall portions (4) of the containers (2) are embraced at least partially to accomplish said friction, wherein the receptacles have a polygonal cross-section, in particular a square-shaped or hexagonal cross-section, wherein the frictional engagement is accomplished by the interaction of opposing side walls of said receptacles and the cylindrical side walls (4) of the containers.

14. The process according to claim 13, wherein all side walls of the openings or receptacles can be adjusted in a coordinated manner all-together between a first position and a second position, wherein, in the first position, the containers can be inserted with little force into the openings or receptacles or can be displaced therein, and wherein, in the second position, the containers are fixed by friction.

15. An apparatus for the treatment or processing of containers (2), which serve for storing substances for medical, pharmaceutical or cosmetic applications or contain such substances, comprising
a conveying device; and
a plurality of processing stations, wherein the respective containers (2) are treated or processed in or at a respective processing station;
said apparatus being configured for the treatment or processing of the containers such that a plurality of containers (2) are conveyed automatically, by means of said conveying device, to said processing stations or pass them to be treated or processed while being supported by a carrier (25; 134) in a regular two-dimensional array, said carrier having a plurality of openings or receptacles (32; 39; 87; 120), which together determine the regular array; and
comprising a crimping device (180), where a metal lid is crimped on the upper rim of the containers,
**characterized in that** said apparatus is configured such that the containers
are displaced in a longitudinal direction to a raised position while being in the respective opening or receptacle to be crimped and are rotated by a turntable about a longitudinal axis in said raised position while the containers are accommodated in the openings or receptacles of the carrier, and
are pushed back into the openings or receptacles after said crimping.

16. The apparatus according to claim 15, which is further configured such that the treatment or processing of the containers is carried out at or in the respective processing station while being supported by a carrier, wherein at least some of the processing stations are preferably operated under sterile environmental conditions.

## Revendications

1. Un procédé de traitement de récipients (2), servant au stockage de substances pour des applications cosmétiques, pharmaceutiques ou médicales ou contenant de telles substances, dans lequel les récipients sont automatiquement convoyés, au moyen d'un convoyeur, à des stations de traitement ou à leur passage, procédé dans lequel
une pluralité de récipients (2) sont convoyés au moyen du convoyeur alors qu'il sont soutenus par un support (25 ; 134) suivant une matrice bidimensionnelle régulière, dans lequel le porteur comporte une pluralité d'ouvertures ou réceptacles (32; 39; 87; 120), délimitant la matrice régulière, et
le traitement des récipients dans une ou au moins une des stations de traitement alors qu'ils sont soutenus par ledit porteur ou pendant qu'ils sont logés dans les ouvertures ou réceptacles dudit transporteur,
**caractérisé en ce qu'**au sein du procédé , on sertit un couvercle métallique sur le bord supérieur des récipients au moyen d'un dispositif de sertissage (180), dans lequel
les récipients sont déplacés dans l'ouverture ou le réceptacle respectif suivant une direction axiale vers une position relevée pour le sertissage et tournant par un plateau tournant autour d'un axe longitudinal dans la position relevée alors que les récipients sont logés dans les ouvertures ou les réceptacles du transporteur, et
sont repoussés dans les ouvertures ou réceptacles pour être soutenus par le transporteur.

2. Le procédé selon la revendication 1 dans lequel, pour assurer le sertissage, les récipients sont levés dans la position relevée au moyen d'une tige de levage (185), dans lequel la table de rotation respective (194) est soutenu sur la tige de levage et dans lequel, durant la rotation, l'on centre le bord supérieur (6) des récipients (2) avec le couvercle métallique (195) y disposé grâce à un disque de centrage (191).

3. Le procédé selon l'une quelconque des revendications précédentes, dans lequel les récipients (2) sont déplacés dans leur ouverture ou réceptacle respective suivant une direction longitudinale vers une position relevée pour être traités ou transformés à la station de traitement.

4. Le procédé selon la revendication 3, dans lequel les récipients continuent à être maintenus en position relevée dans les ouvertures ou réceptacles du transporteur pour être traités ou transformés à la station de traitement..

5. Le procédé selon la revendication 3, dans lequel les récipients continuent à être logés dans les ouvertures ou réceptacles en position relevée du transporteur, et sont cependant soutenus par une surface de support supplémentaire ou par un dispositif de maintien ou de saisie additionnel pour être traités ou transformés à la station de traitement.

6. Le procédé selon la revendication 5, dans lequel la surface de support supplémentaire comporte au moins une table de rotation et d'entraînement sur laquelle les récipients sont mis en rotation alors qu'ils sont traités ou transformés à la station de traitement et alors qu'il sont logés dans les ouvertures ou réceptacles du transporteur.

7. Le procédé selon l'une quelconque des revendications précédentes, dans lequel les supports (25 ; 134) sont retenus dans des récipients de transport et de conditionnement (1) durant traitement ou transformation dans au moins une station de traitement.

8. Le procédé selon l'une quelconque des revendications précédentes, dans lequel des moyens de maintien sont associés aux ouvertures ou réceptacles du transporteur, qui permettent le maintien de la pluralité de récipients (2) sur le transporteurs dans des positions prédéterminées et au sein de la matrice bidimensionnelle, dans lequel les moyens de maintien soutiennent les récipients (2) sur le support (25 ; 134) par liaison de forme.

9. Le procédé de la revendication 8, dans lequel le support (25) comporte une pluralité de nervures transversale mutuellement associées (165), parallèles les unes aux autres et disposées à des intervalles réguliers les unes par rapport aux autres, le long desquelles est disposée une pluralité de bras de maintien (166) de forme concave et élastique en liaison de forme avec les bords supérieurs (6) des récipients logés par une paire de bras de maintien opposés (166), dans lequel les récipients sont insérés ou rétractés des bras de maintien durant leur étalement.

10. Le procédé de la revendication 8, dans lequel les moyens de maintien comprennent au moins deux languettes de maintien (140) qui sont disposées sur le bord d'une ouverture ou d'un réceptacle respectif et faisant saillie vis à vis d'un bord supérieur du support (134) pour le maintien du récipient respectif, dans lequel lesdites languettes de maintien (140) sont pivotées ou pliées de manière élastique lorsque les récipients sont insérés dans les ouvertures ou réceptacles et dans lequel lesdites languettes de maintien (140) maintiennent les récipients avec un jeu radial, dans lequel les languettes de maintien (140) sont de préférence des languettes de maintien élastique.

11. Le procédé de la revendication 10, dans lequel les languettes de maintien (140) soutiennent les récipients (2) reposant de manière lâche sur les bords supérieurs des languettes de maintien (140) avec un bord supérieur (6) formé à une extrémité supérieure des récipients, ou
dans lequel les languettes de maintien (140) sont en prise autour d'un bord élargi (6) formé à une extrémité supérieure des récipients de telle façon que les récipients sont soutenus par les languettes de maintien avec un jeu radial ou avec un jeu radial et axial, dans lequel, de préférence, des surfaces d'insertions penchée (140d) sont formées aux extrémités supérieures des languettes de retenue (140), chacune d'entre elles passant dans un bec de maintien (140e) faisant saillie à l'intérieur des languettes de maintien pour le support des récipients.

12. Le procédé selon l'une quelconque des revendications 1 à 6, dans lequel les moyens de maintien sont associés aux ouvertures ou réceptacles du support, maintenant la pluralité de récipients (2) sur le support à des positions prédéterminés et au sein de la matrice à deux dimensions, dans lequel lesdits moyens de maintien sont configurés pour maintenir par friction les récipients (2) sur le support (25 ; 134).

13. Le procédé de la revendication 12, dans lequel les moyens de maintien sont configurés sous forme de récipients circulaire (39, 65) s'étendant suivant une direction longitudinale des récipients en dans lequel des parties latérales (4) des récipients (2) sont au moins partiellement entourés pour assurer ladite friction, dans lequel les réceptacles présente une section polygonale, en particulier une section en forme carrée ou hexagonale, dans lequel la friction est réalisée par l'interaction de parois latérales opposées desdits réceptacles et des parois cylindriques (4) des récipients.

14. Le procédé selon la revendication 13, dans lequel toutes les parois latérales des ouvertures ou réceptacles peuvent être ajustées ensemble d'une manière coordonnée, entre une première position et une seconde position, dans lequel , dans la première position, les récipients peuvent être insérés à l'intérieur des ouvertures ou réceptacles grâce à une force légère ou peuvent y être déplacés, et dans lequel, dans la seconde position, les récipients sont immobilisés par friction.

15. Un dispositif pour le traitement de récipients (2), servant au stockage de substances pour des applications cosmétiques, pharmaceutiques ou médicales ou contenant de telles substances, ledit dispositif comprenant :
un convoyeur ; et
une pluralité de station de traitement, dans desquelles les récipients respectifs (2) sont traités ou transformés dans une station de traitement respective ;
ledit appareil étant configuré pour le traitement ou la transformation des récipients de telle façon qu'une pluralité de récipients (2) sont automatiquement convoyés, au moyen dudit convoyeur, aux dites stations de traitement ou les traversent pour être traités ou transformés alors qu'il sont soutenus par un support (25 ; 134) dans une matrice bidimensionnelle régulière, ledit porteur ayant une pluralité d'ouvertures ou réceptacles (32; 39; 87; 120), délimitant ensemble la matrice régulière, et
un dispositif de sertissage (180), dans lequel un couvercle métallique est serti sur le bord supérieur des récipients ;
**caractérisé en ce que** le dispositif est configuré de telle façon que les récipients
sont déplacés suivant une direction longitudinale vers une position relevée alors qu'il sont dans l'ouverture ou le réceptacle respectifs pour y être serti et tournent autour d'un axe longitudinal vers la position relevée au moyen d'un plateau tournant alors que les récipients sont logés dans les ouvertures ou les réceptacles du support, et
sont repoussés dans les ouvertures ou réceptacles à la suite du sertissage.

16. Le dispositif de la revendication 15, qui est configuré en outre de telle manière que le traitement des récipients est réalisé à la station de traitement respective alors qu'ils sont soutenus par un support, dans lequel au moins une des stations de traitement est de préférence opérée dans un environnement stérile.
